# EUROPEAN PATENT APPLICATION

(11) **EP 1 666 595 A1**
(43) Date of publication of application: **07.06.2006**
(21) Application number: 05076966.0
(22) Date of filing: 26.10.2001
(51) Int. Cl.: C12N 15/12, C07K 14/47, C07K 16/18, A01K 67/027, C12N 15/11, A61K 31/70

(54) **GAB2 (P97) gene and methods of use thereof**

(30) Priority: 26.10.2000 US 243495 P
(62) Divisional of application: 01994203.6
(71) Applicant: Beth Israel Deaconess Medical Center, Inc., Boston, MA 02215 (US)
(72) Inventor: Gu, Haihua, Needham, MA 02492 (US); Neel, Benjamin G., Wayland, MA 01778 (US); Kinet, Jean-Pierre, Lexington, MA 02421 (US)
(74) Representative: Kirkham, Nicholas Andrew

(57) **Abstract**

This invention relates to the purification, cloning and characterization of a novel gene, Gab2. In response to extracellular stimuli (e.g., cyokines, growth factors, hormones and antigens), Gab2 binds several signal relay molecules, including the protein-tyrosine phosphatase SHP-2 and phosphatidylinositol-3-OH kinase (Pl-3K), which results in the initiation of multiple signaling cascades. Gab2 nucleic acid molecules, peptides, vectors, host cells, probes, antibodies, knockout and transgenic animals are provided. The invention also relates to methods of diagnosis, prevention and treatment of Gab2-mediated conditions such as allergic responses, neoplastic disorders and immune disorders

## Description

### RELATED APPLICATIONS

This applications claims the benefit of U.S. Provisional Application No. 60/243,495, filed October 26, 2000, the teachings of which are incorporated herein by reference in their entirety.

### GOVERNMENT SUPPORT

The invention was supported, in whole or in part, by a grants R01 DK50693, P01 DK50654, R01 DK50654 and R01 CA49152 from the National Institutes of Health. The Government has certain rights in the invention.

### BACKGROUND OF THE INVENTION

Extracellular stimuli are involved in a number of biological processes including cellular proliferation and differentiation. Several components in such biological processes (e.g., signaling cascades) remain unidentified. Thus, there is a need to identify new components of signaling cascades as well as to develop new, improved and effective methods to identify components of signaling cascades.

Allergies are a major medical problem with significant morbidity and even occasional mortality. Current anti-allergy drugs include primarily anti-histamines, cromolyn sodium, and steroids. Steroids can have undesirable side effects. Cromolyn sodium is mainly useful for chronic prevention and often is an irritant in its own right. Antihistamines block the effects of mast cell degranulation, but do not prevent degranulation itself. There is a large market for new anti-allergy agents, as evinced by the success of non-sedating antihistamines when they were introduced several years ago.

Cancer progression is a multi-step process, which evolves from the accumulation of mutations and the deregulation of the genes involved in cell-growth control. In the case of human breast cancer, commonly observed genetic abnormalities include the loss of heterozygosity (LOH) in tumor suppressor genes and the DNA amplification and/or overexpression of growth promoting oncogenes (e.g., c-myc, cyclin D1, and ErbB2/Neu). Breast carcinogenesis due to amplification of ErbB2 as a result of Gab2 overexpression may provide not only a new diagnostic marker for breast cancer detection but also an alternative therapeutic strategy for treating some breast tumors. Since Gab2 knockout mice are essentially healthy except for defects in allergic response, intervention to specifically lower the expression of Gab2 *in vivo* should have minimal side effects.

### SUMMARY OF THE INVENTION

The invention relates to the isolation, cloning, sequencing and characterization of the Gab2 gene or a fragment, derivative or mutation thereof. The present invention also relates to DNA molecules (also referred to herein as DNA sequences or nucleic acid sequences) which encode a protein which comprises Gab2. The present invention also relates to DNA sequences capable of hybridizing to the DNA sequence of Gab2.

The present invention further relates to an expression vector comprising the nucleic acid molecule of Gab2, or a fragment, derivative or mutation thereof. The present invention also relates to a host cell which has been transformed, or transfected, with the expression vector comprising the nucleic acid molecule of Gab2, or a fragment, derivative or mutation thereof.

The protein, or peptides, of the present invention can be used to produce antibodies, both polyclonal and monoclonal, which are reactive with (i.e., bind to) the Gab2 protein, and can be used in diagnostic assays to determine the presence or type of a Gab2-mediated, e.g., Gab2-dependent, disease.

The invention further relates to a transgenic non-human mammal (e.g., a mouse) with a disruption of the Gab2 gene in its genome, either a homozygous disruption or a heterozygous disruption, such that the mammal lacks, or has reduced levels of, functional Gab2 protein. The invention also relates to a transgenic non-human mammal (e.g., a mouse) in which the genome has been altered such that the mammal has increased levels of functional Gab2 protein. These transgenic mammals would exhibit an altered responsiveness to cytokine, growth factor, hormone or antigen stimulation.

The invention further relates to the use of an agent which inhibits a Gab2 interaction with an associated protein, in response to an extracellular stimulus (e.g., a cytokine, growth factor, hormone or antigen) for the manufacture of a medicament for preventing or treating a GAb2-mediated injury (e.g., an allergic response, a neoplastic disease, or an immune disorder).

The agent of the present invention can be selected from the group consisting of proteins, polypeptides, antibodies, oligonucleotides, small molecules, natural product inhibitors, mutants of Gab2, and mutants of Gab2-associated molecules, or wherein the agent is an oligonucleotide antisense to the nucleic acid sequence of Gab2, or antisense to a Gab2 homolog, fragment, complementary sequence, or mutant.

The invention further relates to the nasal, topical or systemic administration of the agent in which the agent is a mutant Gab2, or fragment thereof, which competes with Gab2 for interaction with its associated proteins, or the agent inhibits the expression of Gab2 or the agent inhibits the tyrosyl phosphorylation of Gab2. The invention also relates to the administration of the agent as an insert in a gene therapy vector.

The agent of the present invention can also be employed to inhibit the response of mast cells to FceRI receptor stimulation by administration of the agent to the mast cells. In particular, the agent can prevent a Gab2-mediated injury, for example, an allergic response, by inhibiting a Gab2 interaction with an associated protein in response to an extracellular signal and, thus, prevent activation of a Gab2-mediated signaling cascade.

The agent of the present invention can also be employed to inhibit a neoplastic disease (e.g., leukemia, prostate cancer and breast cancer) by inhibiting a Gab2 interaction with an associated protein in response to an extracellular signal and, thus, prevent activation of a Gab2-mediated signaling cascade. The Gab2 nucleic acid sequence of the present invention can be used to produce a probe that can detect upregulation of Gab2 product in a patient with a neoplastic disorder.

The invention also relates to a method of identifying a drug to that can be administered to treat a Gab2-mediated condition by producing a mouse that is a model of the condition, and administering to the mouse a drug to be assessed for its effectiveness in treating or preventing the condition. If the drug reduces the extent to which the condition is present or progresses, the drug is a drug to be administered to treat the condition.

The present invention also relates to isolated RNA molecules (double-stranded; single-stranded) which mediate RNAi of Gab2. That is, the isolated RNAs of the present invention mediate degradation of Gab2 mRNA. It is not necessary that there be perfect correspondence of the sequences, but the correspondence must be sufficient to enable the RNA to direct RNAi cleavage of the Gab2 mRNA. In a particular embodiment, the RNA molecules of the present invention comprise a 3' hydroxyl group.

The present invention also relates to RNA produced by the methods of the present invention, as well as to RNAs, produced by other methods, such as chemical synthesis or recombinant DNA techniques, that have the same or substantially the same sequences as naturally-occurring RNAs that mediate Gab2 RNAi, such as those produced by the methods of the present invention. The invention further relates to uses of the RNAs, such as for therapeutic or prophylactic treatment and compositions comprising RNAs that mediate Gab2 RNAi, such as pharmaceutical compositions comprising RNAs and an appropriate carrier (e.g., a buffer or water).

The present invention also relates to a method of mediating RNA interference of Gab2 mRNA of a gene in a cell or organism (e.g., mammal such as a mouse or a human). In one embodiment, RNA which targets the Gab2 mRNA is introduced into the cell or organism. The cell or organism is maintained under conditions under which degradation of the mRNA occurs, thereby mediating RNA interference of the Gab2 mRNA in the cell or organism. As used herein, the term "cell or organism in which RNAi occurs" includes both a cell or organism in which Gab2 RNAi occurs as the cell or organism is obtained, or a cell or organism that has been modified so that RNAi occurs.

The present invention also relates to a method for knocking down (partially or completely) the Gab2 gene, thus providing an alternative to presently available methods of knocking down (or out) the Gab2 gene. This method of knocking down gene expression can be used therapeutically or for research (e.g., to generate models of disease states, to examine the function of a gene, to assess whether an agent acts on a gene, to validate targets for drug discovery). In those instances in which gene function is eliminated, the resulting cell or organism can also be referred to as a knockout. One embodiment of the method of producing knockdown cells and organisms comprises introducing into a cell or organism in which Gab2 is to be knocked down, RNA that targets the Gab2 gene and maintaining the resulting cell or organism under conditions under which RNAi occurs, resulting in degradation of the Gab2 mRNA, thereby producing knockdown cells or organisms. Knockdown cells and organisms produced by the present method are also the subject of this invention.

The present invention also encompasses a method of treating a disease or condition associated with the presence of the Gab2 protein in an individual comprising administering to the individual RNA which targets the Gab2 mRNA for degradation. As a result, the protein is not produced or is not produced to the extent it would be in the absence of the treatment.

Also encompassed by the present invention is a method of identifying target sites within a Gab2 mRNA that are particularly suitable for RNAi as well as a method of assessing the ability ofRNAs to mediate RNAi of Gab2.

### BRIEF DESCRIPTION OF THE DRAWINGS

Figure 1 is a schematic of the HPLC of Lys-C fragments from Gab2. Nine peaks (numbered) were selected for Edman sequencing.
Figure 2 depicts the predicted protein sequence derived from Gab2 cDNA (SEQ ID NO: 5). The locations of 8 of the 9 peptides obtained by Edman sequencing are underlined. PH domain is in bold, tyrosine-containing motifs are indicated (- -), and PXXP motifs are in bold.
Figure 3 is a schematic illustration comparing Dos, Gab2 (p97) and Gab1. Percentage sequence identity among family members is indicated. (P)=potential proline-rich domains.
Figure 4 depicts the alignment of Gab/Dos family PH domains (top) and the MBD region in Gab1 and Gab2 (p97)(bottom). β1 and β2 sheets corresponding to the PH domain of PLCδ1 are indicated. Basic amino acid residues between β1 and β2 are in bold. A proposed consensus that may specify Gab/Dos family members is indicated. Two PXXP motifs within Gab1 are overlined.

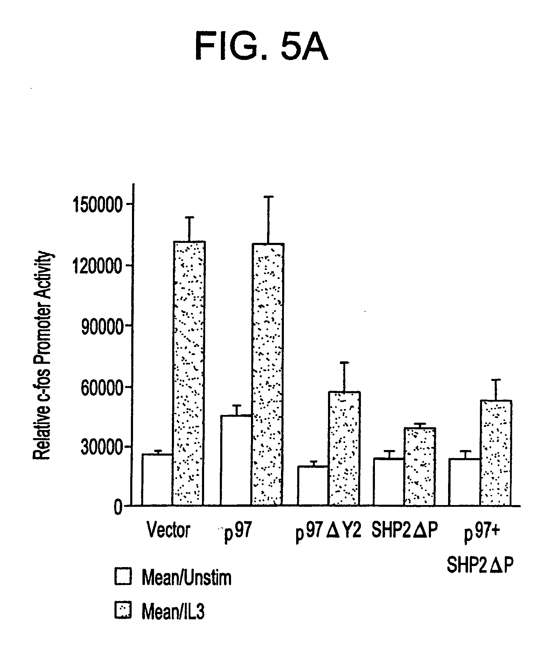

Figure 5A - Figure 5E depict the effects of the SHP-2/Gab2 complex on IL-3-induced transactivation, and IL-3-induced MAPK activation. (A) Effects of expression of Gab2ΔY2HA on IL-3-induced c-fos promoter activity. (B) Effects of Gab2/ΔY2HA on IL-3 induced c-fos promoter activity. (C) Effects of point mutations in Gab2 on IL-3-induced c-fos promoter activity. (D) Normalized GAL-4 luciferase activity of BaF3 cells transiently co-transfected with the indicated expression vectors or vector alone, together with a GAL4-Elk-1 construct and GAL-4- and TK-Renilla luciferase reporters. (E) Effects of Gab2/SHP-2 on STAT-mediated activation.
Figure 6 depicts the nucleotide sequence of Gab2 (SEQ ID NO: 6).
Figure 7 depicts the targeting strategy for the generation of Gab2 knockout mice.
Figure 8 depicts surface expression of Fcε RI in Gab2-/- BMMCs.
Figure 9 depicts Fcε RI-mediated degranulation in Gab2-/- BMMCs.
Figure 10 depicts Fcε RI-evoked TNFα and IL-6 gene expression in Gab2-/BMMCs.
Figure 11 depicts passive cutaneous anaphylaxis in wild-type (WT) and Gab2-/mice.
Figure 12 depicts a schematic illustration of the Gab2 response to Fcε RI receptor stimulation.

### DETAILED DESCRIPTION OF THE INVENTION

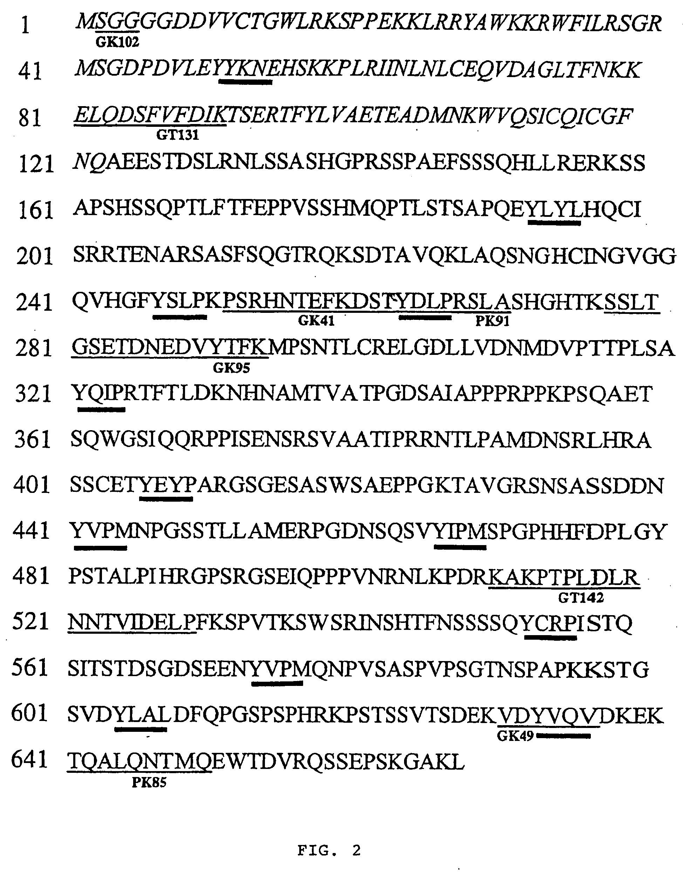

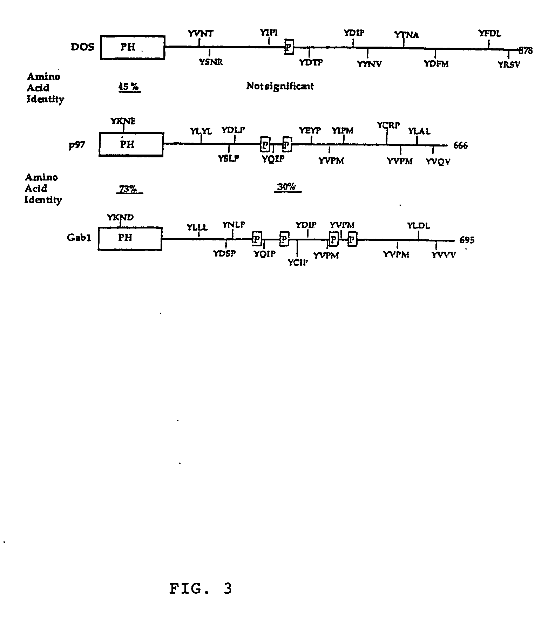

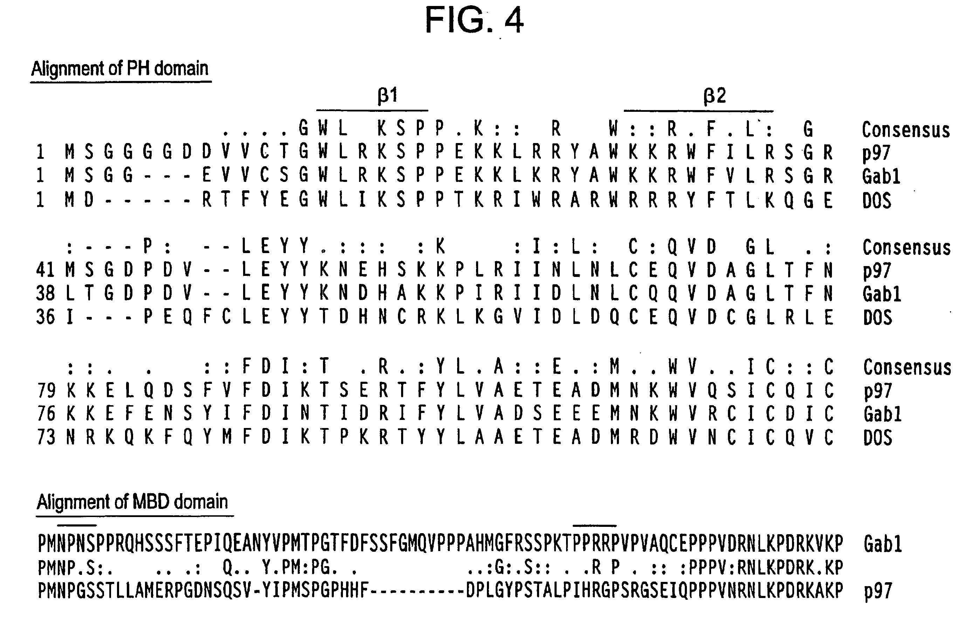

Gab2, a novel member of the DOS/Gab 1 subfamily of scaffolding molecules, was purified, cloned and characterized. DOS/Gab 1 Family members contain an N-terminal PH (Pleckstrin homology) domain, proline-rich motifs and multiple tyrosines, but show minimal sequence identity, mainly in their PH domains (Figures 2, 4) (see Rameh, L. E. et al., J. Biol. Chem. 272, 22059-22066 (1997)). Most of their tyrosyl residues, including those required for SHP-2 binding (Table 2 and Figure 3), occur at similar relative positions and within similar sequence contexts (Figure 3). This suggests that Gab/Dos proteins bind similar signal relay molecules, which may have to bind in a specific orientation. IRS proteins and FRS-2 have similar topography, but are distinguished from Dos/Gab proteins by their PH domains and the order and sequence contexts of their tyrosines and proline-rich motifs. Members of the family include *Drosophila* Dos and mammalian Gab1. Tissue-specific differences in Gab1 and Gab2 expression exist, and at least one functionally important region of Gab1, the MBD, is divergent in Gab2. Although Gab2 and Gab 1 are only distantly related and have distinct functions *in vivo,* the novel protein is termed Gab2 to simplify the nomenclature. Gab2 is also known as p97 and p97/Gab2.

Extracellular stimuli, such as cytokines, growth factors, hormones and antigens, regulate cell proliferation and differentiation via changing the tyrosine phosphorylation states of proteins, such as Gab2, inside the cell. Extracellular receptors, such as cytokine receptors, trigger multiple signaling cascades, which regulate cell proliferation and differentiation. Binding of a cytokine to its cognate receptor activates receptor-associated Janus family (e.g., Jak/Tyk) protein-tyrosine kinase(s) (PTKs), resulting in their phosphorylation and the phosphorylation of receptor cytoplasmic domains. Phosphorylation creates docking sites for SH2 domain-containing signal relay molecules, most of which are substrates for Janus PTKs. Signal relay molecules promote activation of downstream cascades, including the Ras/Raf/mitogen-activated protein Kinase (MAPK), phosphatidylinositol-3' kinase (PI-3K) and Stat cascades. Ultimately, these cascades evoke the transcription of immediate-early genes, such as c-fos (see Thle, J. N. et al., Stem Cells *15*, 105-111 (1997)). Receptor tyrosine kinases (RTKs) and multi-chain immune recognition receptors (MIRRs) utilize analogous signaling strategies.

Gab2 is widely expressed in a variety of tissues, and contains multiple potential serine/threonine phosphorylation sites. Upon cytokine, growth factor, hormone or antigen receptor stimulation, Gab2 becomes tyrosine phosphorylated, most likely by the associated Janus PTK, and involved in the activation of multiple signaling cascades via interaction with different intracellular signaling molecules. Upon tyrosine phosphorylation, Gab2 recruits SH2-containing downstream signal molecules. Gab2 may also transmit extracellular stimuli through interaction with cellular proteins containing SH3 or WW domains. In addition, Gab2 may exert its effect through an interaction with intracellular lipids.

In various hematopoietic cell lines, in response to cytokine stimulation and engagement of MTRRs, Gab2 becomes associated with various SH2-containing molecules, including SHP-2, p85 (the regulatory subunit of PI-3K) and Shc. Gab2, via its interaction with SHP-2, is required for cytokine (e.g., IL-3) induced c-fos gene transcription via a novel mechanism that is parallel to or downstream of MAPK Figures 5A-E). However, Gab2 can also signal to the MAPK cascade under some conditions. This signal does not require SHP-2 binding since Gab2 mutants which do not bind SHP-2 potentiate (do not inhibit) MAPK activation. Conceivably, Gab2 may transmit signals to the MAPK cascade via PI-3K.

In several systems, PI-3K functions downstream of Ras and upstream of MAPK, particularly under conditions of limiting receptor stimulation. The Gab2/p85 complex is critical for PI-3K activation evoked by cytokine receptors, such as IL-3/GM-CSF and IL-2, which do not contain p85-binding sites. However, Gab2 is also tyrosyl phosphorylated upon stimulation of receptors that bind p85 directly (e.g., CSF-1R) or have other means of p85 recruitment (e.g., CD19 for the BCR). In these systems, Gab2 may amplify PI-3K activation.

Upon cytokine stimulation, Gab2 can be recruited to receptor complexes. PH domains bind to phosphatidylinositol lipids, providing a potential recruitment mechanism. Alternatively, the Gab2 region corresponding to the Gab1 MBD may direct binding. A third possibility is that recruitment is indirect. Mutation of Y577 in the IL-3 receptor βc chain, the Shc binding site, severely diminishes Gab2 tyrosyl phosphorylation (see Itoh, T. et al., J. Biol. Chem. *271,* 7587-7592 (1996)). Since Grb2 (presumably via its SH3 domains) binds Gab2 constitutively, and the Grb2 SH2 domain binds to tyrosyl phosphorylated Shc, Gab2 recruitment to the IL-3 receptor may occur via a Shc-Grb2 complex.

### ROLE OF Gab2 IN ALLERGIC RESPONSES

Interaction of multivalent antigen with IgE-bound mast cells provokes several effects: the immediate release of preformed granules containing vasoactive amines including histamine and serotonin (degranulation), secretion of lipid mediators, and the late synthesis and release of cytokines. Fcε RI is the high affinity receptor for IgE. Fcε RI consists of a ligand binding α chain, one β, and two γ chains. Crosslinking Fcε RI by the multivalent antigen activates the β chain associated with tyrosine kinase, lyn. Activated lyn phosphorylates the tyrosine-based activation motifs (IAMTs) in the cytoplasmic domains of the β and γ chains, which recruit and activate tyrosine kinase Syk. Subsequently, lyh and syk phosphorylate various signal relay molecules, resulting in the activation of multiple downstream signaling cascades including phosphatidylinositol 3-kinase (PI-3K) and three major subfamilies of mitogen-activated protein kinases (MAPKs), Erk, JNK, and p38. The lipid products of PI-3K are required for full activation of Tec family tyrosine kinase Btk/Emt, and subsequent phosphorylation and activation of PLCγ. Activated PLCγ then converts PI4,5P2 into inosital 1,4,5-triphosphate (IP3) and diacylglycerol (DAG), which can increase intracellular Ca⁺⁺ level and activate PKC respectively. Both Ca⁺⁺ and PKC are required for the optimal degranulation. Activation of Erk, JNK, and p38 are important for the late phase cytokine production.

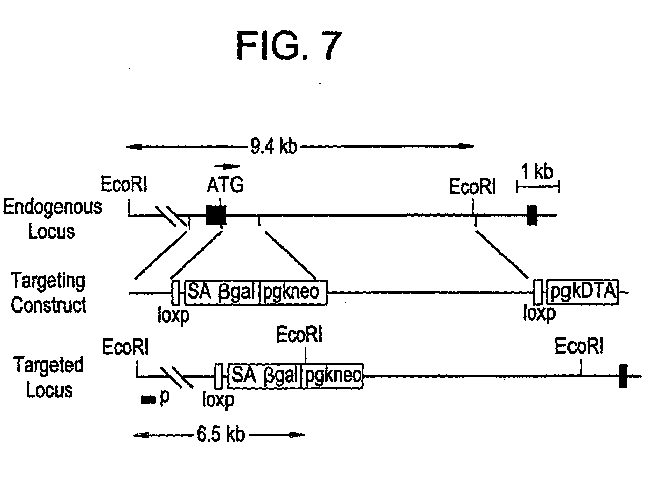

Since one of the major functions of mast cells is involved in IgE-associated immune responses, the role of Gab2 in signaling evoked by Fcε RI in Bone Marrow-derived Mast Cells (BMMCS) was determined. Gab2 knockout mice were generated by conventional gene targeting methods (Figure 7). Bone marrow mast cells or macrophages were isolated from these mice by standard techniques. To examine Fcε signaling, mast cells were loaded with anti-DNP IgE and then stimulated with a range of concentrations of DNP, and serotonin release was quantified. For assessment of macrophage Fcγ responses, bone marrow macrophages were presented with opsonized RBCs, and phagocytosis was quantified by microscopy. In addition, various biochemical parameters were monitored by standard techniques.

In contrast to Gab1 knockout mice, which are embryonic lethal, Gab2 homozygous (-/-) knockout mice are healthy, fertile and appear to live a normal life span. However, analysis of mast cells from Gab2-/- mice show that they are refractory to stimulation through IgE receptors which are the main receptors mediating allergic responses. Mast cell numbers are also diminished in Gab2-/- mice, and Fc gamma receptor signaling in macrophages is defective. Thus, elimination of Gab2 expression, or Gab2 interaction with key signaling molecules, is a powerful approach to prevention of allergic responses.

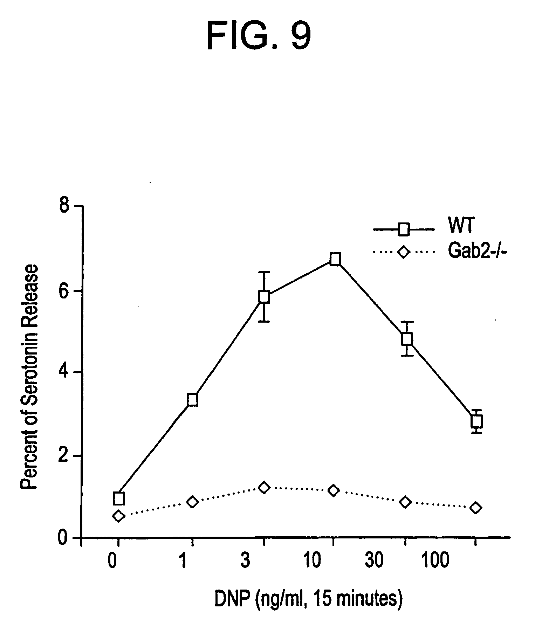

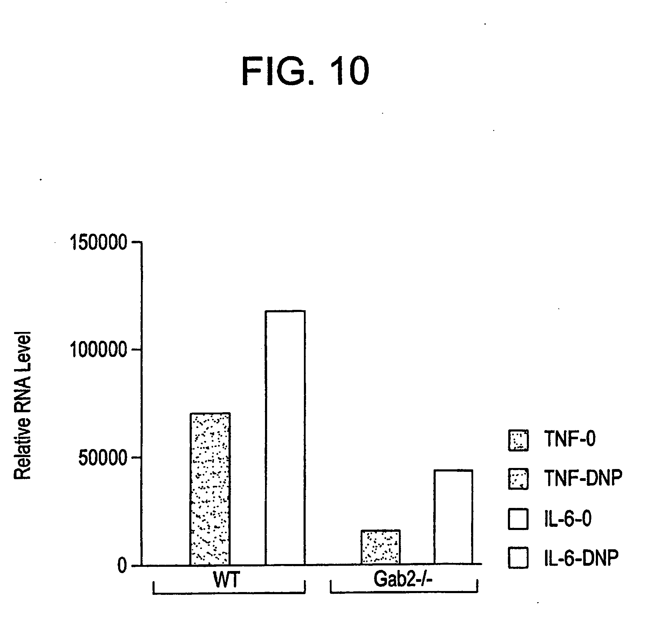

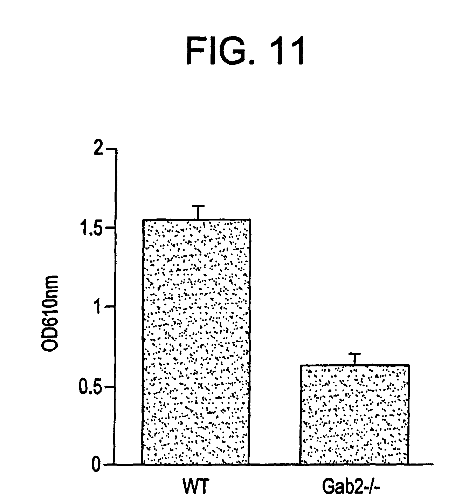

The mast cell degranulation evoked by Fcε RI crosslinking was drarnatically impaired (e.g., a 3-7 fold impairment of Fee-evoked serotonin release) in Gab2-/BMMCS compared to wild type BMMCS (Figure 9). Fcε RI-evoked PLCγ phosphorylation and interleukin-3-induced AKT activation also are significantly diminished in Gab2-/- BMMCS, as well as Fcε RI evoked TNFα and IL-6 gene expression (Figure 10). Furthermore, activation of JNK and p38 are defective in Gab2-/- upon Fcε RI crosslinking. In addition, Gab2-/- mice show decreased passive cutaneous anaphylaxis compared to wild mice (Figure 11). These data show that Gab2 is required for selective events downstream of Fcε activation, most likely those dependent on PI-3K activation.

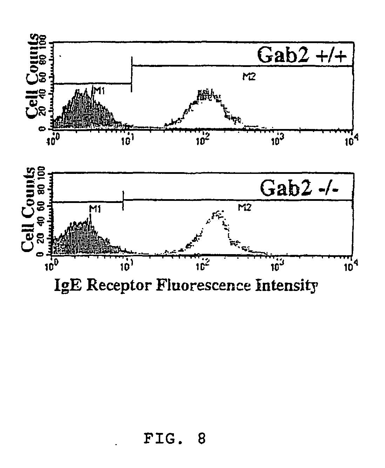

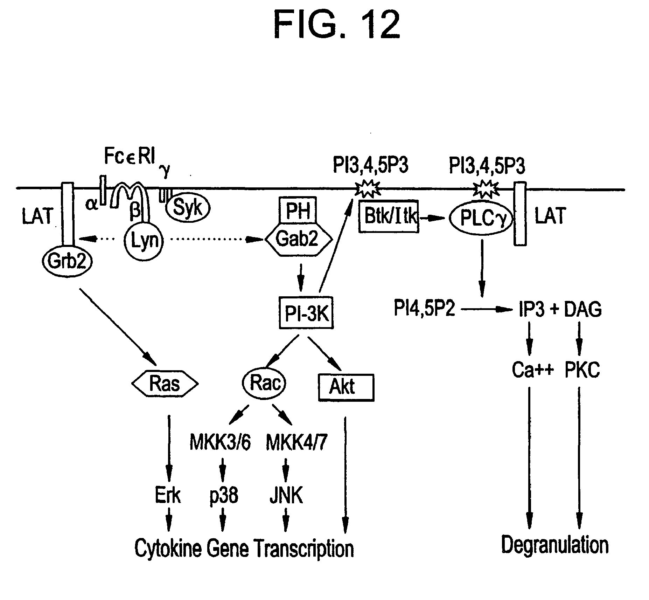

These defects in Fcε RI-evoked biological response and signaling cascades in Gab2-/- BMMCS are not due to a change in surface expression of Fcε RI (Figure 8) or defective activation of upstream tyrosine kinases. Notably, early events in Fcε responses, including surface expression of the IgE receptor, Syk phosphorylation and, most likely, LAT phosphorylation, as well as Erk MAPK activation, are normal. Furthermore, total tyrosyl phosphorylation in Gab2-/- BMMCs is normal upon FcεRI engagement. Moreover, expression of the wild type Gab2 in Gab2-/- BMMCS can rescue the FcεRI-evoked signaling defects. Thus, these data demonstrate that Gab2 plays an important role in FcεRI-evoked signaling and effector function in mast cells and is essential for the IgE-initiated effector function of mast cells by activating the PI-3K/Akt and JNK and p38 cascades (Figure 12). Similar results are obtained in macrophages, where Fcγ-evoked phagocytosis is impaired by up to 50%. Gab2 and its associated signaling molecules represent new targets for developing drugs to treat allergy.

### ROLE OF Gab2 INTERACTION WITH SHP-2

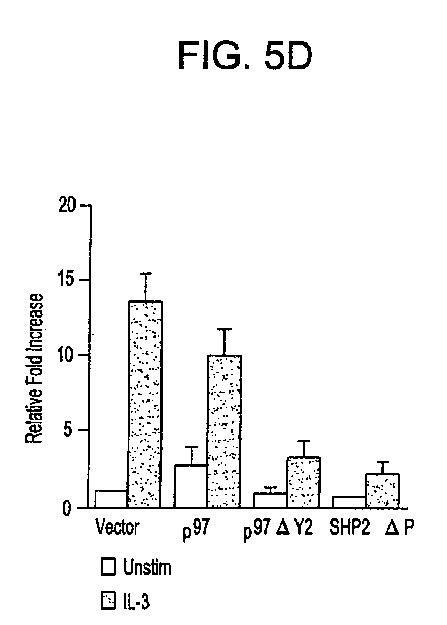

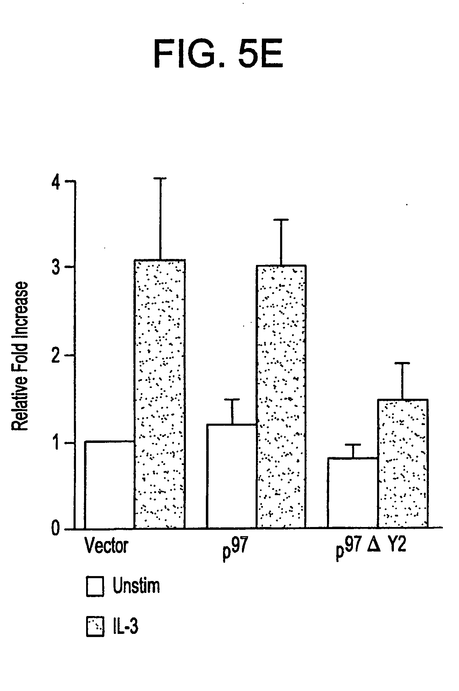

In another embodiment, the Gab2/SHP-2 complex also has a distinct and novel signaling role, since Gab2 mutants inhibit activation of c-fos luciferase (Figure 5A) and Elk (i.e, TCF)- (Figure 5D) and STAT- (Figure 5E) driven reporters. The N-SH2 of SHP-2 binds to tyrosyl phosphorylated Gab2, and N-SH2 engagement activates the enzyme (see Barford, D. and Neel, B. G., Structure *6,* 249-254 (1998)). Dominant negative SHP-2 blocks Gab2 potentiation of basal c-fos promoter activity (Figure 5A), suggesting that upon binding to Gab2, activated SHP-2 dephosphorylates one or more targets to permit c-fos activation. The identity of this target(s) remains unknown. Previous experiments with "substrate trapping" mutants suggested that Gab2 is a SHP-2 substrate, and Dos reportedly is a substrate of Csw. However, mutating its SHP-2 binding sites does not increase Gab2 tyrosyl phosphorylation. Conceivably, the increased association of the cystein to serine mutant (C>S) of SHP-2 with tyrosyl phosphorylated Gab2 is due to SHP-2 regulation of a PTK that phosphorylates Gab2, with consequently increased SHP-2 binding via its SH2 domains to Gab2. However, it remains possible that one or more sites on Gab2 are SHP-2 targets. SHP-2 could dephosphorylate its own binding sites. Alternatively, it could target other sites, but the net increase in their phosphorylation might be less than the decreased phosphorylation due to loss of the SHP-2 sites. If sites on Gab2 are not the primary target(s) of the Gab2/SHP-2 complex, then presumably Gab2 directs SHP-2 to the proper location for accessing its target(s).

Although Gab2 is strongly tyrosyl phosphorylated in response to many stimuli, its association with SHP-2 varies, suggesting that Gab2 may be phosphorylated on distinct sites in response to different stimuli. The widespread expression of Gab2 suggests that its involvement in growth factor and/or cytokine signaling cascades in non-hematopoietic cells should be investigated.

SHP-2 is a critical component of multiple signaling cascades. Embryonic stem cells expressing mutant SHP-2 exhibit defective *ex vivo* hematopoietic differentiation. SHP-2 is required for cytokine-induced MAPK activation. For example, SHP-2 is required for interleukin-5 (IL-5)-induced MAPK activation in eosinophils, interleukin-2 (IL-2)-induced MAPK activation in T cells, and for immediate-early gene activation in response to multiple cytokines in various cellular contexts. RTK and T cell receptor (TCR)-evoked MAPK activation also require SHP-2, and SHP-2 function is necessary for RTK-induced c-fos expression acting, at least in part, to control the activity of the transcription factor Elk-1. Likewise, the *Drosophila* homolog of SHP-2, Csw, is required for RTK-induced gene induction. These studies suggest that SHP-2 is a required positive component of cytokine, RTK, and MIRR signaling cascades, acting upstream of MAPK, which, in turn, lies upstream of immediate-early genes.

However, several lines of evidence raise questions about this simple linear model. For example, SHP-2 has been reported to act both upstream and downstream of Ras. Genetic analysis indicates that Csw acts upstream and downstream of Ras or functions in a parallel cascade in Sevenless signaling. Biochemical and genetic studies suggest that Csw binds to, dephosphorylates, and signals through the Daughter of Sevenless (Dos) gene product, a scaffolding protein remotely related to mammalian Gab1. SHP-2 binds directly to some growth factor receptors, but in other cascades, it binds to scaffolding molecules such as IRS family members, Gab 1, and FRS-2, and/or to one or more transmembrane glycoproteins, such as SHPS-1/SIRPs.

Accordingly, upon cytokine, growth factor, hormone or antigen receptor (B cell receptor/T cell receptor) stimulation, Gab2 becomes tyrosyl phosphorylated and associates with several SH2 domain-containing proteins, including SHP-2. Gab2 via interaction with SHP-2 is required for cytokine induced gene expression, via a novel signaling cascade. Expressed Gab2 mutants that were unable to bind SHP-2 blocks cytokine-induced c-fos promoter activation, inhibiting Elk-1-mediated and STAT5-mediated transactivation, indicating that Gab2 function is required for cytokine-induced immediate early gene activation. In addition, dominant negative SHP-2 inhibits IL-3-induced MAPK activation in BaF3 cells. However, SHP-2 need not bind to Gab2 to participate in MAPK activation, whereas it must bind to Gab2 to allow transcriptional activation (Figures 5A-E). Thus, SHP-2 has at least two sites of action in cytokine signaling. The requirement of SHP-2 for MAPK activation could be mediated through another binding protein, e.g, p135 in BaF3 cells (see Gu, H. et al., J. Biol. Chem. 272, 16421-16430 (1997)), or through the reported direct interaction of SHP-2 and Jak-2 (see Fuhrer, D. K. et al., J. Biol. Chem. 270, 24826-24830 (1995); Ali, S. et al., EMBO J *15*, 135-142 (1996)), whereas the work herein shows that the requirement of SHP-2 for transactivation is mediated by Gab2. SHP-2 action at multiple steps may help explain some of the discrepancies between earlier studies over the site of action of SHP-2 in RTK signaling. Future experiments can be directed to elucidating Gab-2-dependent and -independent functions of SHP-2 in signaling by cytokines, growth factors, and MIRRs. How these interactions culminate in MAPK activation and/or induction of gene expression is unclear. To understand how SHP-2 functions requires both the identification and characterization of its binding proteins and substrates.

Gab2 is an important new regulator of receptor signaling that controls a novel cascade to immediate-early gene activation and suggest new functions for SHP-2 in cytokine receptor signaling. Moreover, since SHP-2 itself is required for IL-3-induced MAPK activation, these data show that SHP-2 is required for at least two steps in cytokine signal transduction, one upstream of, and the other downstream of or parallel to MAPK.

Gab2/SHP-2 may be required for MAPK translocation because MAPKs probably must translocate to the nucleus to phosphorylate transcription factors (e.g., Elk-1) (see Brunet, A. and Pouyssegur, J., Essays Biochem 32, 1-16 (1997)). Consistent with this model, MAPK activation also is required for IL-3-driven STAT reporter activity in BaF3 cells (see Rajotte, D. et al., Blood 88, 2906-2916 (1996)).

Gab2/SHP2 may control a cascade that inhibits dephosphorylation of SRF, TCF, and/or other components of the c-fos transcriptional machinery, perhaps by controlling the serine-threonine kinase KSR. Recent work suggests that KSR activates a serine-threonine phosphatase that catalyzes Elk-1 dephosphorylation. Interestingly, IRS-1 also signals to c-fos without affecting MAPK. Data suggest that this signal may be sent via LRS-1/SHP-2 complexes; by inference, similar signaling cascades may exist for other scaffolding protein/SHP-2 complexes. Whereas Gab2/SHP-2 complex formation appears to be required for full activity of the c-fos promoter, Gab2 mutants unable to bind SHP-2 only partially inhibit c-fos activation. It is unclear whether partial inhibition is due to incomplete interference with endogenous Gab2 by the Gab2 mutants or instead indicates that Gab2/SHP-2 is predominantly an "amplifier" of c-fos activation. The latter is likely because the effects of the Gab2 mutants on c-fos promoter activity are enhanced at lower levels of IL-3. Moreover, the results described herein do not exclude important roles for Gab2 and/or SHP-2 in cascades other than those leading to c-fos promoter activation.

### ROLE OF Gab2 IN NEOPLASTIC DISORDERS

Overexpression of Gab2 may also play a role in neoplastic disorders. It may promote breast carcinogenesis, for example, by amplifying EGFR/ErbB2 initiated growth and survival signals. ErbB2/Neu is the best known gene that is amplified and overexpressed in human breast cancer. Although oncogenic mutant forms of ErbB2 are rarely found in humans, ErbB2 amplification and overexpression has been identified in 20-30% of all the breast cancer cases and is correlated with poor patient prognosis. Interestingly, ErbB2/Neu overexpression is found in ~90% of Ductal Carcinoma *in situ* (DCIS), a malignant ductal carcinoma with an intact basement membrane barrier. Under *in vitro* culture conditions, EGFR(ErbB1) signals have been shown to contribute to disorganized growth of colonies of breast tumor cells under a three-dimensional basement membrane, which mimics DCIS. Furthermore, overexpression of ErbB2 under the control of mouse mammary tumor virus (MMTV) long terminal repeat (LTR) in mammary gland causes mammary tumor formation with fairly long latency in mice. Consistent with a role of ErbB2 in breast cancer, ErbB2 blocking antibodies have been used clinically to reduce regression of some of the ErbB2-overexpressing breast tumors. Collectively, these data indicate that ErbB2/neu overexpression contributes to breast carcinogenesis in human.

ErbB2 belongs to the EGFR/ErbB receptor tyrosine kinase family, which also includes ErbB1/EGFR, ErbB3, and ErbB4. ErbB members can form homodimers or heterodimers depending on binding to specific ligand. Although no ligand for ErbB2 has been identified, ErbB2 acts as a co-receptor for ErbB1, ErbB3, and ErbB4 when the latter binds to EGF family members or neuregulin respectively. Upon ligand binding, ErbB members become dimerized and activated. Activated ErbBs phosphorylate various tyrosine residues in the cytoplasmic domain, resulting in the recruitment and activation of various downstream signaling cascades including ras/raf/MAPK, phosphatidylinositol-3 kinase (PI-3K), and tyrosine phosphatase SHP-2, which provide signals for various cellular responses including proliferation and survival. It is still not clear how each of the ErbB-activated cascades contribute to breast carcinogenesis in *vivo*. Nevertheless, since ErbB2 overexpression inducing breast tumor formation suggests that enhanced PTK activity or ErbB downstream signal relay/adapter components may contribute to breast carcinogenesis.

Although Gab-like molecules have not been implicated in breast cancer previously, overexpression of Gab1 in fibroblasts has been shown to potentiate EGF-mediated cell growth and transformation and anchorage independent growth, and Gab2 is important for cytokine-induced cell growth via its ability to activate PI-3K. Using fluorescence in situ hybridization (FISH) analysis, the Gab2 gene was located on human chromosome 11q13.3-14.2. Since chromosome 11q13 amplification has been found in 10-15% of breast cancer patients, Gab2 expression in breast cancer cell lines and tumors was examined and Gab2 was found to be overexpressed in ~40% of breast cancer cell lines and 20% of primary breast tumor samples tested. Although Gab2 tyrosine phosphorylation and its associated PI-3K activity have been correlated with EGF-induced cell proliferation, the functional evolvement of Gab2 in EGF-mediated cell growth has not been fully investigated.

In one embodiment, the invention encompasses Gab2, its homologs, analogs, variants, mutants, complementary nucleic acid sequences. Encompassed by the present invention are proteins that have substantially the same amino acid sequence as Gab2, or polynucleotides that have substantially the same nucleic acid sequence as the polynucleotides encoding Gab2. "Substantially the same sequence" means a nucleic acid or polypeptide that exhibits at least about 90 % sequence identity with a reference sequence, e.g., another nucleic acid or polypeptide, preferably at least about 95% identity, and more preferably at least about 97% sequence identity with the reference sequence. The length of comparison for sequences will generally be at least 75 nucleotide bases or 25 amino acids, more preferably at least 150 nucleotide bases or 50 amino acids, and most preferably 243-264 nucleotide bases or 81-88 amino acids. "Polypeptide" as used herein indicates a molecular chain of amino acids and does not refer to a specific length of the product. Thus, peptides, oligopeptides and proteins are included within the definition of polypeptide. This term is also intended to include polypeptide that have been subjected to post-expression modifications such as, for example, glycosylations, acetylations, phosphorylations and the like.

"Sequence identity," as used herein, refers to the subunit sequence similarity between two polymeric molecules, *e.g.,* two polynucleotides or two polypeptides. When a subunit position in both of the two molecules is occupied by the same monomeric subunit, *e.g*., if a position in each of two peptides is occupied by serine, then they are identical at that position. The identity between two sequences is a direct function of the number of matching or identical positions, *e.g*., if half *(e.g.,* 5 positions in a polymer 10 subunits in length) of the positions in two peptide or compound sequences are identical, then the two sequences are 50% identical; if 90% of the positions, e.g., 9 of 10 are matched, the two sequences share 90% sequence identity. By way of example, the amino acid sequences R₂R₅R₇R₁₀R₆R₃ and R₉R₈R₁R₁₀R₆R₃ have 3 of 6 positions in common, and therefore share 50% sequence identity, while the sequences R₂R₅R₇R₁₀R₆R₃ and R₈R₁R₁₀R₆R₃ have 3 of 5 positions in common, and therefore share 60% sequence identity. The identity between two sequences is a direct function of the number of matching or identical positions. Thus, if a portion of the reference sequence is deleted in a particular peptide, that deleted section is not counted for purposes of calculating sequence identity, e.g., R₂R₅R₇R₁₀R₆R₃ and R₂R₅R₇R₁₀R₃ have 5 out of 6 positions in common, and therefore share 83.3% sequence identity.

Identity is often measured using sequence analysis software e.g., BLASTN or BLASTP (available at http://www.ncbi.nlm.nih.gov/BLAST/). The default parameters for comparing two sequences (*e.g*., "Blast"-ing two sequences against each other, http://www.ncbi.nlm.nih.gov/gorf/b12.html) by BLASTN (for nucleotide sequences) are reward for match =1, penalty for mismatch = -2, open gap = 5, extension gap = 2. When using BLASTP for protein sequences, the default parameters are reward for match = 0, penalty for mismatch = 0, open gap = 11, and extension gap = 1.

When two sequences share "sequence homology," it is meant that the two sequences differ from each other only by conservative substitutions. For polypeptide sequences, such conservative substitutions consist of substitution of one amino acid at a given position in the sequence for another amino acid of the same class *(e.g.,* amino acids that share characteristics of hydrophobicity, charge, pK or other conformational or chemical properties, *e.g.,* valine for leucine, arginine for lysine), or by one or more non-conservative amino acid substitutions, deletions, or insertions, located at positions of the sequence that do not alter the conformation or folding of the polypeptide to the extent that the biological activity of the polypeptide is destroyed. Examples of "conservative substitutions" include substitution of one non-polar (hydrophobic) residue such as isoleucine, valine, leucine or methionine for another; the substitution of one polar (hydrophilic) residue for another such as between arginine and lysine, between glutamine and asparagine, between threonine and serine; the substitution of one basic residue such as lysine, arginine or histidine for another; or the substitution of one acidic residue, such as aspartic acid or glutamic acid for another; or the use of a chemically derivatized residue in place of a non-derivatized residue; provided that the polypeptide displays the requisite biological activity. Two sequences which share sequence homology may called "sequence homologs."

Homology, for polypeptides, is typically measured using sequence analysis software (e.g., Sequence Analysis Software Package of the Genetics Computer Group, University of Wisconsin Biotechnology Center, 1710 University Avenue, Madison, WI 53705). Protein analysis software matches similar sequences by assigning degrees of homology to various substitutions, deletions, and other modifications. Conservative substitutions typically include substitutions within the following groups: glycine, alanine; valine, isoleucine, leucine; aspartic acid, glutamic acid, asparagine, glutamine; serine, threonine; lysine, arginine; and phenylalanine, tyrosine.

The invention also contemplates mutants of the proteins and peptides disclosed herein, where the mutation(s) do not substantially alter the activity of the protein or peptide, that is the mutations are effectively "silent" mutations.

By "mutant" of Gab2 is meant a polypeptide that includes any change in the amino acid sequence relative to the amino acid sequence of the equivalent reference Gab2 polypeptide. Such changes can arise either spontaneously or by manipulations by man, by chemical energy (e.g., X-ray), or by other forms of chemical mutagenesis, or by genetic engineering, or as a result of mating or other forms of exchange of genetic information. Mutations include, e.g., base changes, deletions, insertions, inversions, translocations, or duplications. Mutant forms of Gab2 may display either increased or decreased activity relative to the equivalent reference Gab2 polynucleotide, and such mutants may or may not also comprise additional amino acids derived from the process of cloning, e.g., amino acid residues or amino acid sequences corresponding to full or partial linker sequences.

Mutants/fragments of the protein of the present invention can be generated by PCR cloning. To make such fragments, PCR primers are designed from known sequence in such a way that each set of primers will amplify known subsequence from the overall protein. These subsequences are then cloned into an appropriate expression vector and the expressed protein tested for its activity as described in the assays described herein.

Mutants/fragments of the protein of the present invention can also be generated by *Pseudomonas* elastase digestion, as described by Mariyama, M. *et al*. (1992, *J. Biol. Chem*. 267:1253-8).

By "analog" of Gab2 is meant a non-natural molecule substantially similar to either the entire Gab2 molecule or a fragment or allelic variant thereof, and having substantially the same or superior biological activity. Such analogs are intended to include derivatives (*e*.*g*., chemical derivatives, as defined above) of the biologically active Gab2, as well as its fragments, mutants, homologs, and allelic variants, which derivatives exhibit a qualitatively similar agonist or antagonist effect to that of the unmodified Gab2 polypeptide, fragment, mutant, homolog, or allelic variant.

By "allele" of Gab2 is meant a polypeptide sequence containing a naturally-occurring sequence variation relative to the polypeptide sequence of the reference Gab2 polypeptide. By "allele" of a polynucleotide encoding the Gab2 polypeptide is meant a polynucleotide containing a sequence variation relative to the reference polynucleotide sequence encoding the reference Gab2 polypeptide, where the allele of the polynucleotide encoding the Gab2 polypeptide encodes an allelic form of the Gab2 polypeptide.

It is possible that a given polypeptide may be either a fragment, a mutant, an analog, or allelic variant of Gab2, or it may be two or more of those things, *e.g*., a polypeptide may be both an analog and a mutant of the Gab2 polypeptide. For example, a shortened version of the Gab2 molecule (*e*.*g*., a fragment of Gab2) may be created in the laboratory. If that fragment is then mutated through means known in the art, a molecule is created that is both a fragment and a mutant of Gab2. In another example, a mutant may be created, which is later discovered to exist as an allelic form of Gab2 in some mammalian individuals. Such a mutant Gab2 molecule would therefore be both a mutant and an allelic variant. Such combinations of fragments, mutants, allelic variants, and analogs are intended to be encompassed in the present invention.

Also encompassed by the present invention are chemical derivatives of Gab2. "Chemical derivative" refers to a subject polypeptide having one or more residues chemically derivatized by reaction of a functional side group. Such derivatized residues include for example, those molecules in which free amino groups have been derivatized to form amine hydrochlorides, p-toluene sulfonyl groups, carbobenzoxy groups, t-butyloxycarbonyl groups, chloroacetyl groups or formyl groups. Free carboxyl groups may be derivatized to form salts, methyl and ethyl esters or other types of esters or hydrazides. Free hydroxyl groups may be derivatized to form O-acyl or O-alkyl derivatives. The imidazole nitrogen of histidine may be derivatized to form N-imbenzylhistidine. Also included as chemical derivatives are those peptides which contain one or more naturally occurring amino acid derivatives of the twenty standard amino acids. For examples: 4-hydroxyproline may be substituted for proline; 5-hydroxylysine may be substitute for lysine; 3-methylhistidine may be substituted for histidine; homoserine may be substituted for serine; and omithine may be substituted for lysine.

The present invention also includes fusion proteins and chimeric proteins comprising the Gab2 protein, its fragments, mutants, homologs, analogs, and allelic variants. A fusion or chimeric protein can be produced as a result of recombinant expression and the cloning process, e.g., the protein may be produced comprising additional amino acids or amino acid sequences corresponding to full or partial linker sequences, comprises additional vector sequence added to the protein, including a HA tag. As used herein, the term "fusion or chimeric protein" is intended to encompass changes of this type to the original protein sequence. A fusion or chimeric protein can consist of a multimer of a single protein, *e.g*., repeats of the Gab2 protein, or the fusion and chimeric proteins can be made up of several proteins. The term "fusion protein" or "chimeric protein" as used herein can also encompass additional components for e.g., delivering a chemotherapeutic agent, wherein a polynucleotide encoding the chemotherapeutic agent is linked to the polynucleotide encoding the Gab2 protein.

Multimeric proteins comprising Gab2, its fragments, mutants, homologs, analogs and allelic variants are also intended to be encompassed by the present invention. By "multimer" is meant a protein comprising two or more copies of a subunit protein. The subunit protein may be the protein of the present invention, e.g., Gab2 repeated two or more times, or a fragment, mutant, homolog, analog or allelic variant, *e.g*., a Gab2 mutant or fragment, repeated two or more times. Such a multimer may also be a fusion or chimeric protein, *e*.*g*., a repeated Gab2 mutant may be combined with polylinker sequence, and/or one or more peptides, *e.g*.,Gab2-associated peptides, which may be present in a single copy, or may also be tandemly repeated, *e.g*., a protein may comprise two or more multimers within the overall protein.

In one embodiment, the invention encompasses Gab2, its homologs, analogs, variants, mutants, complementary nucleic acid sequences, and sequences which can be used to design probes which hybridize to Gab2 or its complementary strand. The design of the probes should preferably follow these parameters: (a) it should be designed to an area of the sequence which has the fewest ambiguous bases ("N's"), if any, and (b) it should be designed to have a Tₘ of approx. 80°C (assuming 2°C for each A or T and 4 degrees for each G or C).

The probes should preferably be labeled with g-³²P-ATP (specific activity 6000 Ci/mmole) and T4 polynucleotide kinase using commonly employed techniques for labeling oligonucleotides. Other labeling techniques can also be used. Unincorporated label should preferably be removed by gel filtration chromatography or other established methods. The amount of radioactivity incorporated into the probe should be quantitated by measurement in a scintillation counter. In one embodiment, specific activity of the resulting probe should be approximately 4 x 10⁶ dpm/pmole. The bacterial culture containing the pool of full-length clones can be thawed and 100 µl of the stock used to inoculate a sterile culture flask containing 25 ml of sterile L-broth containing ampicillin at 100 µg/ml. The culture can be grown to saturation at 37°C, and the saturated culture should preferably be diluted in fresh L-broth. Aliquots of these dilutions can be plated to determine the dilution and volume which will yield approximately 5000 distinct and well-separated colonies on solid bacteriological media containing L-broth containing ampicillin at 100 µg/ml and agar at 1.5% in a 150 mm petri dish when grown overnight at 37°C. Other known methods of obtaining distinct, well-separated colonies can also be employed.

Standard colony hybridization procedures can then be used to transfer the colonies to nitrocellulose filters and lyse, denature and bake them. Highly stringent conditions include those that are at least as stringent as, for example, 1x SSC at 65°C, or 1x SSC and 50% formamide at 42°C. Moderate stringency conditions include those that are at least as stringent as, for example, 4x SSC at 65°C, or 4x SSC and 50% formamide at 42°C. Reduced stringency conditions can include, for example, those that are at least as stringent as 4x SSC at 50°C, or 6x SSC and 50% formamide at 40°C.

The filter is then preferably incubated at 65°C for 1 hour with gentle agitation in 6x SSC (20x stock is 175.3 g NaCl/liter, 88.2 g Na citrate/liter, adjusted to pH 7.0 with NaOH) containing 0.5% SDS, 100 µg/ml of yeast RNA, and 10 mM EDTA (approximately 10 mL per 150 mm filter). The probe can then added to the hybridization mix at a concentration greater than or equal to 1 x 10⁶ dpm/mL. The filter is then preferably incubated at 65°C with gentle agitation overnight. The filter is then preferably washed in 500 mL of 2x SSC/0.5% SDS at room temperature without agitation, preferably followed by 500 mL of 2x SSC/0.1% SDS at room temperature with gentle shaking for 15 minutes. A third wash with 0.1x SSC/0.5% SDS at 65°C for 30 minutes to 1 hour is optional. The filter is then preferably dried and subjected to autoradiography for sufficient time to visualize the positives on the X-ray film. Other known hybridization methods can also be employed. The positive colonies are then picked, grown in culture, and plasmid DNA isolated using standard procedures. The clones can then be verified by restriction analysis, hybridization analysis, or DNA sequencing.

Stringency conditions for hybridization refers to conditions of temperature and buffer composition which permit hybridization of a first nucleic acid sequence to a second nucleic acid sequence, wherein the conditions determine the degree of identity between those sequences which hybridize to each other. Therefore, "high stringency conditions" are those conditions wherein only nucleic acid sequences which are very similar to each other will hybridize. The sequences may be less similar to each other if they hybridize under moderate stringency conditions. Still less similarity is needed for two sequences to hybridize under low stringency conditions. By varying the hybridization conditions from a stringency level at which no hybridization occurs, to a level at which hybridization is first observed, conditions can be determined at which a given sequence will hybridize to those sequences that are most similar to it. The precise conditions determining the stringency of a particular hybridization include not only the ionic strength, temperature, and the concentration of destabilizing agents such as formamide, but also on factors such as the length of the nucleic acid sequences, their base composition, the percent of mismatched base pairs between the two sequences, and the frequency of occurrence of subsets of the sequences (e.g., small stretches of repeats) within other non-identical sequences. Washing is the step in which conditions are set so as to determine a minimum level of similarity between the sequences hybridizing with each other. Generally, from the lowest temperature at which only homologous hybridization occurs, a 1% mismatch between two sequences results in a 1°C decrease in the melting temperature (Tₘ) for any chosen SSC concentration. Generally, a doubling of the concentration of SSC results in an increase in the Tₘ of about 17°C. Using these guidelines, the washing temperature can be determined empirically, depending on the level of mismatch sought. Hybridization and wash conditions are explained in *Current Protocols in Molecular Biology* (Ausubel, F.M. *et al.,* eds., John Wiley & Sons, Inc., 1995, with supplemental updates) on pages 2.10.1 to 2.10.16, and 6.3.1 to 6.3.6.

High stringency conditions can employ hybridization at either (1) 1x SSC (10x SSC = 3 M NaCl, 0.3 M Na₃-citrate·2H₂O (88 g/liter), pH to 7.0 with 1 M HCl), 1% SDS (sodium dodecyl sulfate), 0.1 - 2 mg/ml denatured salmon sperm DNA at 65°C, (2) 1x SSC, 50% formamide, 1% SDS, 0.1 - 2 mg/ml denatured salmon sperm DNA at 42°C, (3) 1% bovine serum albumen (fraction V), 1 mM Na₂-EDTA, 0.5 M NaHPO₄ (pH 7.2) (1 M NaHPO₄ = 134 g Na₂HPO₄·7H₂O, 4 ml 85% H₃PO₄ per liter), 7% SDS, 0.1 - 2 mg/ml denatured salmon sperm DNA at 65°C, (4) 50% formamide, 5x SSC, 0.02 M Tris-HCl (pH 7.6), 1x Denhardt's solution (100x = 10 g Ficoll 400, 10 g polyvinylpyrrolidone, 10 g bovine serum albumin (fraction V), water to 500 ml), 10% dextran sulfate, 1% SDS, 0.1 - 2 mg/ml denatured salmon sperm DNA at 42°C, (5) 5x SSC, 5x Denhardt's solution, 1% SDS, 100 µg/ml denatured salmon sperm DNA at 65°C, or (6) 5x SSC, 5x Denhardt's solution, 50% formamide, 1% SDS, 100 µg/ml denatured salmon sperm DNA at 42°C, with high stringency washes of either (1) 0.3 - 0.1x SSC, 0.1% SDS at 65°C, or (2) 1 mM Na₂EDTA, 40 mM NaHPO₄ (pH 7.2), 1% SDS at 65°C. The above conditions are intended to be used for DNA-DNA hybrids of 50 base pairs or longer. Where the hybrid is believed to be less than 18 base pairs in length, the hybridization and wash temperatures should be 5 - 10°C below that of the calculated Tₘ of the hybrid, where Tₘ in °C = (2 x the number of A and T bases) + (4 x the number of G and C bases). For hybrids believed to be about 18 to about 49 base pairs in length, the Tₘ in °C = (81.5°C + 16.6(log₁₀M) + 0.41(% G + C) - 0.61 (% formamide) - 500/L), where "M" is the molarity of monovalent cations (*e*.*g*., Na⁺), and "L" is the length of the hybrid in base pairs.

Moderate stringency conditions can employ hybridization at either (1) 4x SSC, (10x SSC = 3 M NaCl, 0.3 M Na₃-citrate·2H₂O (88 g/liter), pH to 7.0 with 1 M HCl), 1% SDS (sodium dodecyl sulfate), 0.1 - 2 mg/ml denatured salmon sperm DNA at 65°C, (2) 4x SSC, 50% formamide, 1% SDS, 0.1 - 2 mg/ml denatured salmon sperm DNA at 42°C, (3) 1% bovine serum albumen (fraction V), 1 mM Na₂·EDTA, 0.5 M NaBPO₄ (pH 7.2) (1 M NaHPO₄ = 134 g Na₂HPO₄·7H₂O, 4 ml 85% H₃PO₄ per liter), 7% SDS, 0.1 - 2 mg/ml denatured salmon sperm DNA at 65°C, (4) 50% formamide, 5x SSC, 0.02 M Tris-HCl (pH 7.6), 1x Denhardt's solution (100x = 10 g Ficoll 400, 10 g polyvinylpyrrolidone, 10 g bovine serum albumin (fraction V), water to 500 ml), 10% dextran sulfate, 1% SDS, 0.1 - 2 mg/ml denatured salmon sperm DNA at 42°C, (5) 5x SSC, 5x Denhardt's solution, 1% SDS, 100 µg/ml denatured salmon sperm DNA at 65°C, or (6) 5x SSC, 5x Denhardt's solution, 50% formamide, 1% SDS, 100 µg/ml denatured salmon sperm DNA at 42°C, with moderate stringency washes of 1x SSC, 0.1% SDS at 65°C. The above conditions are intended to be used for DNA-DNA hybrids of 50 base pairs or longer. Where the hybrid is believed to be less than 18 base pairs in length, the hybridization and wash temperatures should be 5 - 10°C below that of the calculated Tₘ of the hybrid, where Tₘ in °C = (2 x the number of A and T bases) + (4 x the number of G and C bases). For hybrids believed to be about 18 to about 49 base pairs in length, the Tₘ in °C = (81.5°C + 16.6(log₁₀M) + 0.41(% G + C) - 0.61 (% formamide) - 500/L), where "M" is the molarity of monovalent cations (e.g., Na⁺), and "L" is the length of the hybrid in base pairs.

Low stringency conditions can employ hybridization at either (1) 4x SSC, (10x SSC = 3 M NaCl, 0.3 M Na₃-citrate·2H₂O (88 g/liter), pH to 7.0 with 1 M HCI), 1% SDS (sodium dodecyl sulfate), 0.1-2 mg/ml denatured salmon sperm DNA at 50°C, (2) 6x SSC, 50% formamide, 1% SDS, 0.1-2 mg/ml denatured salmon sperm DNA at 40°C, (3) 1% bovine serum albumen (fraction V), 1 mM Na₂·EDTA, 0.5 M NaHPO₄ (pH 7.2) (1 M NaHPO₄ = 134 g Na₂BFO₄·7H₂O, 4 ml 85% H₃PO₄ per liter), 7% SDS, 0.1-2 mg/ml denatured salmon sperm DNA at 50°C, (4) 50% formamide, 5x SSC, 0.02 M Tris-HCl (pH 7.6), 1x Denhardt's solution (100x = 10 g Ficoll 400,10 g polyvinylpyrrolidone, 10 g bovine serum albumin (fraction V), water to 500 ml), 10% dextran sulfate, 1% SDS, 0.1 - 2 mg/ml denatured salmon sperm DNA at 40°C, (5) 5x SSC, 5x Denhardt's solution, 1% SDS, 100 µg/ml denatured salmon sperm DNA at 50°C, or (6) 5x SSC, 5x Denhardt's solution, 50% formamide, 1% SDS, 100 µg/ml denatured salmon sperm DNA at 40°C, with low stringency washes of either 2x SSC, 0.1% SDS at 50°C, or (2) 0.5% bovine serum albumin (fraction V), 1 mM Na₂EDTA, 40 mM NaHPO₄ (pH 7.2), 5% SDS. The above conditions are intended to be used for DNA-DNA hybrids of 50 base pairs or longer. Where the hybrid is believed to be less than 18 base pairs in length, the hybridization and wash temperatures should be 5 - 10°C below that of the calculated Tₘ of the hybrid, where Tₘ in °C = (2 x the number of A and T bases) + (4 x the number of G and C bases). For hybrids believed to be about 18 to about 49 base pairs in length, the Tₘ in °C = (81.5°C + 16.6(log₁₀M) + 0.41(% G + C) - 0.61 (% formamide) - 500/L), where "M" is the molarity of monovalent cations *(e.g.,* Na⁺), and "L" is the length of the hybrid in base pairs.

The invention also encompasses the use of wild type or mutant versions of Gab2 as inserts for vectors. Such vectors can be used to produce Gab2 proteins in large quantities. They can also be used for the delivery of nucleic acids to a cell, e.g., a host cell. Such a vector may also bring about the replication and/or expression of the transferred nucleic acid pieces and can be used to produce Gab2 protein in large quantities. Examples of vectors include nucleic acid molecules derived, e.g., from a plasmid, bacteriophage, or a mammalian, plant or insect virus, or non-viral vectors such as ligand-nucleic acid conjugates, liposomes, or lipid-nucleic acid complexes. It may be desirable that the transferred nucleic molecule is operatively linked to an expression control sequence to form an expression vector capable of expressing the transferred nucleic acid. Such transfer of nucleic acids is generally called "transformation," and refers to the insertion of an exogenous polynucleotide into a host cell, irrespective of the method used for the insertion. For example, direct uptake, transduction or f-mating are included. The exogenous polynucleotide may be maintained as a non-integrated vector, for example, a plasmid, or alternatively, may be integrated into the host genome. "Operably linked" refers to a situation wherein the components described are in a relationship permitting them to function in their intended manner, *e.g*., a control sequence "operably linked" to a coding sequence is ligated in such a manner that expression of the coding sequence is achieved under conditions compatible with the control sequence. A "coding sequence" is a polynucleotide sequence which is transcribed into mRNA and translated into a polypeptide when placed under the control of (*e*.*g*., operably linked to) appropriate regulatory sequences. The boundaries of the coding sequence are determined by a translation start codon at the 5'-terminus and a translation stop codon at the 3'-terminus. Such boundaries can be naturally-occurring, or can be introduced into or added the polynucleotide sequence by methods known in the art. A coding sequence can include, but is not limited to, mRNA, cDNA, and recombinant polynucleotide sequences.

The vector into which the cloned polynucleotide is cloned may be chosen because it functions in a prokaryotic, or alternatively, it is chosen because it functions in a eukaryotic organism. Two examples of vectors which allow for both the cloning of a polynucleotide encoding the Gab2 protein, and the expression of this protein from the polynucleotides, are the pET22b and pET28(a) vectors (Novagen, Madison, Wisconsin, USA) and a modified pPICZαA vector (InVitrogen, San Diego, California, USA), which allow expression of the protein in bacteria and yeast, respectively. See for example, WO 99/29878, the entire teachings which are hereby incorporated by reference.

Once a polynucleotide has been cloned into a suitable vector, it can be transformed, or transfected, into an appropriate host cell. By "host cell" is meant a cell which has been or can be used as the recipient of transferred nucleic acid by means of a vector. Host cells can be prokaryotic or eukaryotic, mammalian, plant, or insect, and can exist as single cells, or as a collection, e.g., as a culture, or in a tissue culture, or in a tissue or an organism. Host cells can also be derived from normal or diseased tissue from a multicellular organism, e.g., a mammal. "Host cell", as used herein, is intended to include not only the original cell which was transformed with a nucleic acid, but also descendants of such a cell, which still, comprise, or contain, the nucleic acid.

In one embodiment, the isolated polynucleotide encoding the Gab2 protein additionally comprises a polynucleotide linker encoding a peptide. Such linkers are known to those of skill in the art and, for example the linker can comprise at least one additional codon encoding at least one additional amino acid. Typically the linker comprises one to about twenty or thirty amino acids. The polynucleotide linker is translated, as is the polynucleotide encoding the Gab2 protein, resulting in the expression of a GAb2 protein with at least one additional amino acid residue at the amino or carboxyl terminus of the protein. Importantly, the additional amino acid, or amino acids, do not compromise the activity of the Gab2 protein.

After inserting the selected polynucleotide into the vector, the vector is transformed into an appropriate prokaryotic strain and the strain is cultured *(e.g.,* maintained) under suitable culture conditions for the production of the biologically active GAb2 protein, thereby producing a biologically active Gab2 protein, or mutant, derivative, fragment or fusion protein thereof. In one embodiment, the invention comprises cloning of a polynucleotide encoding a Gab2 protein into the vectors pET22b, pET17b or pET28a, which are then transformed into bacteria. The bacterial host strain then expresses the Gab2 protein.

In another embodiment of the present invention, the eukaryotic vector comprises a modified yeast vector. One method is to use a pPICzα plasmid wherein the plasmid contains a multiple cloning site. The multiple cloning site has inserted into it a HA.Tag motif. Additionally the vector can be modified to add a *Nde*I site, or other suitable restriction sites. Such sites are well known to those of skill in the art.

One method of producing Gab2, for example, is to amplify the polynucleotide of SEQ ID NO:6, and clone it into an expression vector, transform the vector containing the polynucleotide into a host cell capable of expressing the polypeptide encoded by the polynucleotide, culturing the transformed host cell under culture conditions suitable for expressing the protein, and then extracting and purifying the protein from the culture.

In another embodiment, the Gab2 protein may also be expressed as a product of transgenic animals, *e.g.,* as a component of the milk of transgenic cows, goats, sheep or pigs, or as a product of a transgenic plant, *e.g.,* combined or linked with starch molecules in maize. These methods can also be used with a subsequence of SEQ ID NO:6 to produce portions of the protein of SEQ ID NO:5.

Gab2 may also be produced by conventional, known methods of chemical synthesis. Methods for constructing the proteins of the present invention by synthetic means are known to those skilled in the art. The synthetically-constructed Gab2 protein sequence, by virtue of sharing primary, secondary or tertiary structural and/or conformational characteristics with *e.g.,* recombinantly-produced Gab2, may possess biological properties in common therewith, including biological activity. Thus, the synthetically-constructed Gab2 protein sequence may be employed as biologically active or immunological substitute for e.g., recombinantly-produced, purified Gab2 protein in screening of therapeutic compounds and in immunological processes for the development of antibodies.

Polynucleotides encoding Gab2 can be cloned out of isolated DNA or a cDNA library. Nucleic acids and polypeptides referred to herein as "isolated" are nucleic acids or polypeptides substantially free (*i.e*., separated away from) the material of the biological source from which they were obtained (*e.g*., as exists in a mixture of nucleic acids or in cells), which may have undergone further processing. "Isolated" nucleic acids or polypeptides include nucleic acids or polypeptides obtained by methods described herein, similar methods, or other suitable methods, including essentially pure nucleic acids or polypeptides, nucleic acids or polypeptides produced by chemical synthesis, by combinations of chemical or biological methods, and recombinantly produced nucleic acids or polypeptides which are isolated. An isolated polypeptide therefore means one which is relatively free of other proteins, carbohydrates, lipids, and other cellular components with which it is normally associated. An isolated nucleic acid is not immediately contiguous with (*i*.*e*., covalently linked to) both of the nucleic acids with which it is immediately contiguous in the naturally-occurring genome of the organism from which the nucleic acid is derived. The term, therefore, includes, for example, a nucleic acid which is incorporated into a vector (*e.g*., an autonomously replicating virus or plasmid), or a nucleic acid which exists as a separate molecule independent of other nucleic acids such as a nucleic acid fragment produced by chemical means or restriction endonuclease treatment.

An extracellular stimulus is any substance (e.g., a compound such as a molecule) that associates or interacts with a cell, directly or indirectly, such that the association or interaction results in a signaling cascade within the cell. Such extracellular stimuli include cytokines, growth factors, hormones and antigens.

In another embodiment, the biological function of Gab2 can be compromised by inhibitors which can specially block the interaction of Gab2 with its associated molecules. Such inhibitors may be useful in the treatment of numerous disorders, including allergic responses, immunodisorders and cancer. For example, Gab2 is constitutively tyrosine phosphorylated in a variety of cells transformed by BCR-ABL, the oncogene responsible for chronic myelogenous leukemia, as well as by its relative, the TEL-ABL fusion protein, suggesting that deregulation of a cascade involving SHP-2 and Gab2 may contribute to cell transformation. In addition, over-expression and/or constitutive phosphorylation of Gab2 could contribute to other diseases including other neoplastic diseases.

As used herein, the term "inhibitor of Gab2 interaction/function" refers to an agent (e.g., an oligonucleotide, a molecule, a compound, or a protein) which can inhibit a (i.e., one or more) function of Gab2. Inhibition can be partial or complete. For example, an inhibitor of Gab2 function can inhibit Gab2's association with one or more proteins (e.g., SHP-2, p85, Grb2) to Gab2 and/or inhibit signal transduction mediated through Gab2 (e.g., MAPK activation, c-fos gene transcription). Accordingly, Gab2-mediated processes and cellular responses (e.g., proliferation, migration, chemotactic responses, secretion or degranulation (e.g., acute rejection, chronic rejection)) can be inhibited with an inhibitor of Gab2 function. As used herein, "Gab2" refers to naturally occurring Gab2 (including vertebrate Gab2, e.g., mammalian Gab2, such as human *(Homo sapiens)* Gab2) and also encompasses naturally occurring variants, such as allelic variants and splice variants.

Preferably, the inhibitor of Gab2 function is a compound which is, for example, a small organic molecule, natural product, protein (e.g., antibody, cytokine, antigen), peptide or peptidomimetic. Inhibitors of Gab2 function can be identified, for example, by screening libraries or collections of molecules, such as, the Chemical Repository of the National Cancer Institute, as described herein or using other suitable methods.

The term "natural product", as used herein, refers to a compound which can be found in nature, for example, naturally occurring metabolites of marine organisms (e.g., tunicates, algae), plants or other organisms and which possess biological activity, e.g., can inhibit Gab2 function.

Natural products can be isolated and identified by suitable means. For example, a suitable biological source (e.g., vegetation) can be homogenized (e.g., by grinding) in a suitable buffer and clarified by centrifugation, thereby producing an extract. The resulting extract can be assayed for the capacity to inhibit Gab2 function, for example, by the assays described herein. Extracts which contain an activity that inhibit Gab2 function can be further processed to isolate the Gab2 inhibitor by suitable methods, such as, fractionation (e.g., column chromatography (e.g., ion exchange, reverse phase, affinity), phase partitioning, fractional crystallization) and assaying for biological activity. Once isolated the structure of a natural product can be determined (e.g., by nuclear magnetic resonance (NMR)) and those of skill in the art can devise a synthetic scheme for synthesizing the natural product. Thus, a natural product can be isolated (e.g., substantially purified) from nature or can be fully or partially synthetic. A natural product can be modified (e.g., derivatized) to optimize its therapeutic potential. Thus, the term "natural product", as used herein, includes those compounds which are produced using standard medicinal chemistry techniques to optimize the therapeutic potential of a compound which can be isolated from nature.

A "neoplastic disorder", or "Cancer" means neoplastic growth, hyperplastic or proliferative growth or a pathological state of abnormal cellular development and includes solid tumors, non-solid tumors, and any abnormal cellular proliferation, such as that seen in leukemia. As used herein, "cancer" also means Gab2-dependent cancers and tumors, *i. e.,* tumors that require for their growth (expansion in volume and/or mass) an amplification/overexpression of Gab2. "Regression" refers to the reduction of tumor mass and size as determined using methods well-known to those of skill in the art.

The term "neoplastic disease , as used herein, reters to a malignant pathologies involving a Gab2-mediated injury or other malignancies involving Gab2, such as, but not limited to, breast cancer, prostate cancer, carcinomas, such as Ductal Carcinoma in *situ*, and leukemias (acute, chronic myelocytic, chronic lymphocytic and/or myelodyspastic syndrome); and lymphomas (Hodgkin's and non-Hodgkin's lymphomas, such as malignant lymphomas (Burkitt's lymphoma or Mycosis fungoides)).

The term "allergic response", as used herein, refers to inflammatory or allergic diseases and conditions, including, but not limited to, respiratory allergic diseases such as asthma, allergic rhinitis, hypersensitivity lung diseases, hypersensitivity pneumonitis, interstitial lung diseases (ILD) (e.g., idiopathic pulmonary fibrosis, or ILD associated with rheumatoid arthritis, systemic lupus erythematosus, ankylosing spondylitis, systemic sclerosis, Sjogren's syndrome, polymyositis or dermatomyositis); systemic anaphylaxis or hypersensitivity responses, drug allergies (e.g., to penicillin, cephalosporins), insect sting allergies; inflammatory bowel diseases, such as Crohn's disease and ulcerative colitis; spondyloarthropathies; scleroderma; psoriasis and inflammatory dermatoses such as dermatitis, eczema, atopic dermatitis, allergic contact dermatitis, urticaria; vasculitis (e.g., necrotizing, cutaneous, and hypersensitivity vasculitis).

The term "immune disorder", as used herein, refers to any immune disease, including autoimmune diseases including, but not limited to, arthritis (e.g., rheumatoid arthritis, juvenile rheumatoid arthritis, psoriatic arthritis), multiple sclerosis, systemic lupus erythematosus, myasthenia gravis, juvenile onset diabetes, nephritides such as glomerulonephritis, autoimmune thyroiditis, Behcet's disease, graft rejection (e.g., in transplantation); or to other diseases or conditions (including Gab2-mediated diseases or conditions), in which undesirable inflammatory responses are to be inhibited can be treated, including, but not limited to, reperfusion injury, atherosclerosis, certain hematologic malignancies, cytokine-induced toxicity (e.g., septic shock, endotoxic shock), polymyositis, dermatomyositis.

In another embodiment, the nucleic acid sequence of Gab2 of the invention or its homologs, fragments or complementary sequences can be used to design antisense oligonucleotides. Such agents can be administered via a variety of routes, including nasal, systemic or topical. In one embodiment, they can be used in anti-allergy therapy.

The agent (e.g., Gab2 inhibitor, or additional therapeutic agent) can be administered by any suitable parenteral or nonparenteral route, including, for example, topically (e.g., cream, ointment), or nasally (e.g., solution, suspension). Parenteral administration can include, for example, intramuscular, intravenous, intraarticular, intraarterial, intrathecal, subcutaneous, or intraperitoneal administration. The agent (e.g., Gab2 inhibitor, additional therapeutic agent) can also be administered orally (e.g., in capsules, suspensions, tablets or dietary), transdermally, topically, by inhalation (e.g., intrabronchial, intranasal, oral inhalation or intranasal drops) or rectally. Administration can be local or systemic as indicated. The preferred mode of administration can vary depending upon the particular agent (e.g., Gab2 inhibitor, additional therapeutic agent) chosen, however, oral, systemic or parenteral administration is generally preferred.

Delivery can be *in vitro, in vivo,* or *ex vivo.* Delivery can be via a variety of means, including transfection, transformation and electroporation. The cell can be present in a biological sample obtained from the patient (e.g., blood, bone marrow) and used in the treatment of disease such as cancer, immunosuppression/immunostimulation, neurodegeneration or cardiac hypertrophy, or can be obtained from cell culture and used to dissect cell proliferation, cell death or protein degradation cascades in *in vivo* and *in vitro* systems. After contact with the viral vector comprising Gab2 or a Gab2 mutant, the sample can be returned or readministered to a cell or patient according to methods known to those practiced in the art. In the case of delivery to a patient or experimental animal model (e.g., rat, mouse, monkey, chimpanzee), such a treatment procedure is sometimes referred to as *ex vivo* treatment or therapy. Frequently the cell is targeted from the patient or animal and retumed to the patient or animal once contacted with the viral vector comprising the activated mutant of the present invention. *Ex vivo* gene therapy has been described, for example, in Kasid, *et al., Proc. Natl. Acad. Sci. USA 87*:473 (1990); Rosenberg, *et al., New Engl. J. Med. 323*:570 (1990); Williams, *et al., Nature 310*476 (1984); Dick, *et al., Cell 42:*71 (1985); Keller, *et al., Nature 318*:149 (1985) and Anderson, *et al*., U.S. Patent No. 5,399,346 (1994).

Use of timed release or sustained release delivery systems are also included in the invention. Such systems are highly desirable in situations where surgery is difficult or impossible, e.g., patients debilitated by age or the disease course itself, or where the risk-benefit analysis dictates control over cure.

A sustained-release matrix, as used herein, is a matrix made of materials, usually polymers, which are degradable by enzymatic or acid/base hydrolysis or by dissolution. Once inserted into the body, the matrix is acted upon by enzymes and body fluids. The sustained-release matrix desirably is chosen from biocompatible materials such as liposomes, polylactides (polylactic acid), polyglycolide (polymer of glycolic acid), polylactide co-glycolide (co-polymers of lactic acid and glycolic acid) polyanhydrides, poly(ortho)esters, polyproteins, hyaluronic acid, collagen, chondroitin sulfate, carboxylic acids, fatty acids, phospholipids, polysaccharides, nucleic acids, polyamino acids, amino acids such as phenylalanine, tyrosine, isoleucine, polynucleotides, polyvinyl propylene, polyvinylpyrrolidone and silicone. A preferred biodegradable matrix is a matrix of one of either polylactide, polyglycolide, or polylactide co-glycolide (co-polymers of lactic acid and glycolic acid).

The agent (e.g., Gab2 inhibitor, additional therapeutic agent) can be administered as a neutral compound or as a salt or esther. Salts of compounds containing an amine or other basic group can be obtained, for example, by reacting with a suitable organic or inorganic acid, such as hydrogen chloride, hydrogen bromide, acetic acid, perchloric acid and the like. Compounds with a quaternary ammonium group also contain a counteranion such as chloride, bromide, iodide, acetate, perchlorate and the like. Salts of compounds containing a carboxylic acid or other acidic functional group can be prepared by reacting with a suitable base, for example, a hydroxide base. Salts of acidic functional groups contain a countercation such as sodium, potassium and the like.

As used herein, the terms "pharmaceutically acceptable," "physiologically tolerable" and grammatical variations thereof as they refer to compositions, carriers, diluents and reagents, are used interchangeably and represent that the materials are capable of administration to or upon a mammal with a minimum of undesirable physiological effects such as nausea, dizziness, gastric upset and the like. The preparation of a pharmacological composition that contains active ingredients dissolved or dispersed therein is well understood in the art and need not be limited based on formulation. Typically such compositions are prepared as injectables either as liquid solutions or suspensions, however, solid forms suitable for solution, or suspensions, in liquid prior to use can also be prepared. The preparation can also be emulsified.

The inhibitor of Gab2 function can be administered to the individual as part of a pharmaceutical composition comprising a Gab2 inhibitor and a pharmaceutically or physiologically acceptable carrier. Pharmaceutical compositions for co-therapy can comprise an inhibitor of Gab2 function and one or more additional therapeutic agents. An inhibitor of Gab2 function and an additional therapeutic agent can be components of separate pharmaceutical compositions which can be mixed together prior to administration or administered separately. Formulation will vary according to the route of administration selected (e.g., solution, emulsion, capsule).

The active (e.g., therapeutic) ingredient can be mixed with excipients which are pharmaceutically acceptable and compatible with the active ingredient and in amounts suitable for use in the therapeutic methods described herein. Suitable pharmaceutical or physiological carriers can contain inert ingredients which do not interact with the inhibitor of Gab2 function and/or additional therapeutic agent. Standard pharmaceutical formulation techniques can be employed, such as those described in Remington's Pharmaceutical Sciences, Mack Publishing Company, Easton, PA. Suitable carriers, e.g., excipients for parenteral administration, include, for example, sterile water, dextrose, glycerol, ethanol, physiological saline, bacteriostatic saline (saline containing about 0.9% benzyl alcohol), phosphate-buffered saline, Hank's solution, Ringer's-lactate and the like and combinations thereof. Methods for encapsulating compositions (such as in a coating of hard gelatin or cyclodextran) are known in the art (Baker, *et al*., "Controlled Release of Biological Active Agents", John Wiley and Sons, 1986). In addition, if desired, the composition can contain minor amounts of auxiliary substances such as wetting or emulsifying agents, pH buffering agents and the like which enhance the effectiveness of the active ingredient.

As used herein, the term "effective amount" also means the total amount of each active component of the composition or method that is sufficient to show a meaningful patient benefit, *i.e*., treatment, healing, prevention or amelioration of the relevant medical condition, or an increase in rate of treatment, healing, prevention or amelioration of such conditions. For example, an "effective amount" of a Gab2 inhibitor is an amount sufficient to achieve a desired therapeutic and/or prophylactic effect, such as an amount sufficient to inhibit mast cell degranulation. In addition, an effective amount is an amount sufficient to inhibit a (i.e., one or more) function of Gab2 (e.g., Gab2 extracellular signal-induced c-fos activation, and/or Gab2 ligand-induced secretion (e.g. degranulation) of antihistamines), and thereby, inhibit a Gab2-mediated injury (e.g., allergic response, immune disorder, neoplastic disease). An "effective amount" of an additional therapeutic agent is an amount sufficient to achieve a desired therapeutic and/or prophylactic effect. When applied to a combination, the term refers to combined amounts of the active ingredients that result in the therapeutic effect, whether administered in combination, serially or simultaneously.

The amount of agent (e.g., Gab2 inhibitor, additional therapeutic agent) administered to the individual will depend on the characteristics of the individual, such as general health, age, sex, body weight and tolerance to drugs as well as the degree, severity and type of rejection. The skilled artisan will be able to determine appropriate dosages depending on these and other factors.

The dosage of the agent of Gab2 of the present invention will also depend on the disease state or condition being treated along with the above clinical factors and the route of administration of the compound. For treating humans or animals, about 10 mg/kg of body weight to about 20 mg/kg of body weight of the protein can be administered. In combination therapies, *e.g*., the agents of the invention in combination with radiotherapy, chemotherapy, or immunotherapy, it may be possible to reduce the dosage, *e.g*., to about 0.1 mg/kg of body weight to about 0.2 mg/kg of body weight. Depending upon the half life of the agent in the particular animal or human, the agent can be administered between several times per day to once a week. It is to be understood that the present invention has application for both human and veterinary use. The methods of the present invention contemplate single as well as multiple administrations, given either simultaneously or over an extended period of time. In addition, the agent can be administered in conjunction with other forms of therapy, *e.g*., chemotherapy, radiotherapy, or immunotherapy.

The term "unit dose" when used in reference to a therapeutic composition of the present invention refers to physically discrete units suitable as unitary dosage for the subject, each unit containing a predetermined quantity of active material calculated to produce the desired therapeutic effect in association with the required diluent; *i.e.,* carrier or vehicle. Preferred unit dosage formulations are those containing a daily dose or unit, daily sub-dose, or an appropriate fraction thereof, of the administered ingredient. It should be understood that in addition to the ingredients, particularly mentioned above, the formulations of the present invention may include other agents conventional in the art having regard to the type of formulation in question. Optionally, cytotoxic agents may be incorporated or otherwise combined with the agent, or biologically functional protein fragments thereof, to provide dual therapy to the patient.

In addition, Gab2 nucleic acid sequences (e.g., cDNA sequence) can be used to identify relevant downstream targets of Gab2, for example, those required for allergic responses. Moreover, Gab2 nucleic acid sequences (e.g., cDNA sequence) along with critical binding molecule(s), can be used to screen for small molecule and/or natural product inhibitors of the interaction(s).

Conversely, defects in Gabz expression and/or sequence may contribute to other diseases, such as immunodeficiency. Thus, the Gab2 coding sequence may also be useful for screening in various diseases, and in gene therapy applications.

In another embodiment, the novel scaffolding molecule Gab2 can be used to identify inhibitors of PH domain/lipids; Gab2/receptor (e.g., cytokine, growth factor, hormone and antigen receptor); Gab2/SH2 or SH3 protein interactions. Thus, inhibitors which can block Gab2 interaction with other intracellular molecules via PH domain, SH2, and SH3 domain can be potentially useful for immunosuppression and cancer therapy. Nucleic acid probes for Gab2 can also be used to detect upregulation or downregulation of Gab2 product in specimens from patients with leukemia such as CML, or to generate antibodies to detect changes in Gab2 expression or phosphorylation, for example, in patients with various neoplastic states.

RNA interference or "RNAi" is a term initially coined by Fire and co-workers to describe the observation that double-stranded RNA (dsRNA) can block gene expression when it is introduced into worms (Fire et al., Nature 391, 806-811 (1998)). dsRNA directs gene-specific, post-transcriptional silencing in many organisms, including vertebrates, and is a tool for studying gene function.

RNA interference can be used as a method for knocking down (partially or completely) (out) Gab2. This method of knocking out Gab2 gene expression can be used therapeutically or for research (e.g., to generate models of disease states, to examine the function of a Gab2, to assess whether an agent acts on a Gab2, to validate targets for drug discovery). In those instances in which Gab2 function is eliminated, the resulting cell or organism can also be referred to as a knockout. One embodiment of the method of producing knockdown cells and organisms comprises introducing into a cell or organism in which Gab2 is to be knocked down, RNA of sufficient length that targets Gab2 and maintaining the resulting cell or organism under conditions under which RNAi occurs, resulting in degradation of the mRNA of Gab2, thereby producing knockdown cells or organisms. Gab2 knockdown cells and organisms produced by the present method are also the subject of this invention.

Furthermore, RNAi can be used as a method of examining or assessing the function of Gab2 in a cell or organism. In one embodiment, RNA of sufficient length which targets mRNA of Gab2 is introduced into a cell or organism in which RNAi occurs. The cell or organism is referred to as a test cell or organism. The test cell or organism is maintained under conditions under which degradation of Gab2 mRNA occurs. The phenotype of the test cell or organism is then observed and compared to that of an appropriate control cell or organism, such as a corresponding cell or organism that is treated in the same manner except that Gab2 is not targeted. A difference between the phenotypes of the test and control cells or organisms provides information about the function of the degraded Gab2 mRNA. The information provided may be sufficient to identify (define) the function of Gab2 or may be used in conjunction with information obtained from other assays or analyses to do so.

Moreover, RNAi can be used as a method of validating whether an agent acts on Gab2. In this method, RNA of sufficient length that targets the Gab2 mRNA is introduced into a cell or organism in which RNAi occurs. Whether the agent has an effect on the cell or organism is determined.

In addition, RNAi can be used as a method of validating whether Gab2 is a target for drug discovery or development. RNA of sufficient length that targets Gab2 is introduced into a cell or organism. The cell or organism is maintained under conditions in which degradation of the Gab2 mRNA occurs, resulting in decreased expression of Gab2. Whether decreased expression of Gab2 has an effect on the cell or organism is determined, wherein if decreased expression of Gab2 has an effect, then the Gab2 product is a target for drug discovery or development.

RNAi can also be used as a method of treating a disease or condition associated with the presence of Gab2 protein in an individual comprising administering to the individual RNA of sufficient length which targets the mRNA of Gab2 (the mRNA that encodes the protein) for degradation. As a result, the protein is not produced or is not produced to the extent it would be in the absence of the treatment. Techniques for using such methods are found in PCT Application Number PCT/US01/10188 (WO 01/75164), the contents of which are incorporated by reference herein in their entirety.

The invention also encompasses genetically manipulated cell and animals, including knockout and transgenic mice, such as Gab2-/- mice or mice which overexpress Gab2 such as Whey Acidic Promoter- Gab2 (WAP-Gab2) transgene mice as described herein.

In another embodiment, vectors described herein can be useful in a gene therapy setting, whereby a polynucleotide encoding the Gab2 protein, integrins, integrin subunits, or a mutant, fragment, or fusion protein thereof, is introduced and regulated in a patient. Various methods of transferring or delivering DNA to cells for expression of the gene product protein, otherwise referred to as gene therapy, are disclosed in *Gene Transfer into Mammalian Somatic Cells in vivo,* N. Yang, *Crit. Rev. Biotechn.* 12(4):335-356 (1992), which is hereby incorporated in its entirety by reference. Gene therapy encompasses incorporation of DNA sequences into somatic cells or germ line cells for use in either *ex vivo* or *in vivo* therapy. Gene therapy functions to replace genes, augment or inhibit normal or abnormal gene function, and to combat infectious diseases and other pathologies.

Strategies for treating these medical problems with gene therapy include therapeutic strategies such as identifying the defective gene and then adding a functional gene to either replace or inhibit the function of the defective gene or to augment a slightly functional gene; or prophylactic strategies, such as adding a gene for the product protein that will treat the condition or that will make the tissue or organ more susceptible to a treatment regimen.

Many protocols for transfer of the DNA or regulatory sequences of the Gab2 protein are envisioned in this invention. Transfection of promoter sequences, other than one normally found specifically associated with the Gab2 protein, or other sequences which would increase production of the Gab2 protein are also envisioned as methods of gene therapy.

Gene transfer methods for gene therapy fall into three broad categories: physical *(e.g.,* electroporation, direct gene transfer and particle bombardment), chemical *(e.g.,* lipid-based carriers, or other non-viral vectors) and biological (e.g., virus-derived vector and receptor uptake). For example, non-viral vectors may be used which include liposomes coated with DNA. Such liposome/DNA complexes may be directly injected intravenously into the patient. It is believed that the liposome/DNA complexes are concentrated in the liver where they deliver the DNA to macrophages and Kupffer cells. These cells are long lived and thus provide long term expression of the delivered DNA. Additionally, vectors or the "naked" DNA of the gene may be directly injected into the desired organ, tissue or tumor for targeted delivery of the therapeutic DNA.

Gene therapy methodologies can also be described by delivery site. Fundamental ways to deliver genes include *ex vivo* gene transfer, *in vivo* gene transfer, and *in vitro* gene transfer. In *ex vivo* gene transfer, cells are taken from the patient and grown in cell culture. The DNA is transfected into the cells, the transfected cells are expanded in number and then reimplanted in the patient. In *in vitro* gene transfer, the transformed cells are cells growing in culture, such as tissue culture cells, and not particular cells from a particular patient. These "laboratory cells" are transfected, the transfected cells are selected and expanded for either implantation into a patient or for other uses.

*In vivo* gene transfer involves introducing the DNA into the cells of the patient when the cells are within the patient. Methods include using virally mediated gene transfer using a noninfectious virus to deliver the gene in the patient or injecting naked DNA into a site in the patient and the DNA is taken up by a percentage of cells in which the gene product protein is expressed. Additionally, the other methods described herein, such as use of a "gene gun," may be used for *in vitro* insertion of the DNA or regulatory sequences controlling production of the Gab2 protein.

Chemical methods of gene therapy may involve a lipid based compound, not necessarily a liposome, to transfer the DNA across the cell membrane. Lipofectins or cytofectins, lipid-based positive ions that bind to negatively charged DNA, make a complex that can cross the cell membrane and provide the DNA into the interior of the cell. Another chemical method uses receptor-based endocytosis, which involves binding a specific ligand to a cell surface receptor and enveloping and transporting it across the cell membrane. The ligand binds to the DNA and the whole complex is transported into the cell. The ligand gene complex is injected into the blood stream and then target cells that have the receptor will specifically bind the ligand and transport the ligand-DNA complex into the cell.

Many gene therapy methodologies employ viral vectors to insert genes into cells. For example, altered retrovirus vectors have been used in *ex vivo* methods to introduce genes into peripheral and tumor-infiltrating lymphocytes, hepatocytes, epidermal cells, myocytes, or other somatic cells. These altered cells are then introduced into the patient to provide the gene product from the inserted DNA.

Viral vectors have also been used to insert genes into cells using *in vivo* protocols. To direct the tissue-specific expression of foreign genes, WAP-acting regulatory elements or promoters that are known to be tissue-specific can be used. Alternatively, this can be achieved using *in situ* delivery of DNA or viral vectors to specific anatomical sites *in vivo.* For example, gene transfer to blood vessels *in vivo* was achieved by implanting *in vitro* transduced endothelial cells in chosen sites on arterial walls. The virus infected surrounding cells which also expressed the gene product. A viral vector can be delivered directly to the in *vivo* site, by a catheter for example, thus allowing only certain areas to be infected by the virus, and providing long-term, site specific gene expression. *In vivo* gene transfer using retrovirus vectors has also been demonstrated in mammary tissue and hepatic tissue by injection of the altered virus into blood vessels leading to the organs.

Viral vectors that have been used for gene therapy protocols include but are not limited to, retroviruses, other RNA viruses such as poliovirus or Sindbis virus, adenovirus, adeno-associated virus, herpes viruses, SV 40, vaccinia and other DNA viruses. Replication-defective murine retroviral vectors are the most widely utilized gene transfer vectors. Murine leukemia retroviruses are composed of a single strand RNA complexed with a nuclear core protein and polymerase (pol) enzymes, encased by a protein core (gag) and surrounded by a glycoprotein envelope (env) that determines host range. The genomic structure of retroviruses include the *gag, pol,* and env genes enclosed at by the 5' and 3' long terminal repeats (LTR). Retroviral vector systems exploit the fact that a minimal vector containing the 5' and 3' LTRs and the packaging signal are sufficient to allow vector packaging, infection and integration into target cells providing that the viral structural proteins are supplied in trans in the packaging cell line. Fundamental advantages of retroviral vectors for gene transfer include efficient infection and gene expression in most cell types, precise single copy vector integration into target cell chromosomal DNA, and ease of manipulation of the retroviral genome.

The adenovirus is composed of linear, double stranded DNA complexed with core proteins and surrounded with capsid proteins. Advances in molecular virology have led to the ability to exploit the biology of these organisms to create vectors capable of transducing novel genetic sequences into target cells *in vivo.* Adenoviral-based vectors will express gene product proteins at high levels. Adenoviral vectors have high efficiencies of infectivity, even with low titers of virus. Additionally, the virus is fully infective as a cell free virion so injection of producer cell lines is not necessary. Another potential advantage to adenoviral vectors is the ability to achieve long term expression of heterologous genes *in vivo.*

Mechanical methods of DNA delivery include fusogenic lipid vesicles such as liposomes or other vesicles for membrane fusion, lipid particles of DNA incorporating cationic lipid such as lipofectin, polylysine-mediated transfer of DNA, direct injection of DNA, such as microinjection of DNA into germ or somatic cells, pneumatically delivered DNA-coated particles, such as the gold particles used in a "gene gun," and inorganic chemical approaches such as calcium phosphate transfection. Particle-mediated gene transfer methods were first used in transforming plant tissue. With a particle bombardment device, or "gene gun," a motive force is generated to accelerate DNA-coated high density particles (such as gold or tungsten) to a high velocity that allows penetration of the target organs, tissues or cells. Particle bombardment can be used in *in vitro* systems, or with *ex vivo* or *in vivo* techniques to introduce DNA into cells, tissues or organs. Another method, ligand-mediated gene therapy, involves complexing the DNA with specific ligands to form ligand-DNA conjugates, to direct the DNA to a specific cell or tissue.

It has been found that injecting plasmid DNA into muscle cells yields high percentage of the cells which are transfected and have sustained expression of marker genes. The DNA of the plasmid may or may not integrate into the genome of the cells. Non-integration of the transfected DNA would allow the transfection and expression of gene product proteins in terminally differentiated, non-proliferative tissues for a prolonged period of time without fear of mutational insertions, deletions, or alterations in the cellular or mitochondrial genome. Long-term, but not necessarily permanent, transfer of therapeutic genes into specific cells may provide treatments for genetic diseases or for prophylactic use. The DNA could be reinjected periodically to maintain the gene product level without mutations occurring in the genomes of the recipient cells. Non-integration of exogenous DNAs may allow for the presence of several different exogenous DNA constructs within one cell with all of the constructs expressing various gene products.

Electroporation for gene transfer uses an electrical current to make cells or tissues susceptible to electroporation-mediated mediated gene transfer. A brief electric impulse with a given field strength is used to increase the permeability of a membrane in such a way that DNA molecules can penetrate into the cells. This technique can be used in *in vitro* systems, or with *ex vivo* or *in vivo* techniques to introduce DNA into cells, tissues or organs.

Carrier mediated gene transfer *in vivo* can be used to transfect foreign DNA into cells. The carrier-DNA complex can be conveniently introduced into body fluids or the bloodstream and then site-specifically directed to the target organ or tissue in the body. Both liposomes and polycations, such as polylysine, lipofectins or cytofectins, can be used. Liposomes can be developed which are cell specific or organ specific and thus the foreign DNA carried by the liposome will be taken up by target cells. Injection of immunoliposomes that are targeted to a specific receptor on certain cells can be used as a convenient method of inserting the DNA into the cells bearing the receptor. Another carrier system that has been used is the asialoglycoportein/polylysine conjugate system for carrying DNA to hepatocytes for *in vivo* gene transfer.

The transfected DNA may also be complexed with other kinds of carriers so that the DNA is carried to the recipient cell and then resides in the cytoplasm or in the nucleoplasm. DNA can be coupled to carrier nuclear proteins in specifically engineered vesicle complexes and carried directly into the nucleus.

Gene regulation of the Gab2 may be accomplished by administering compounds that bind to the Gab2 gene, or control regions associated with the Gab2 gene, or its corresponding RNA transcript to modify the rate of transcription or translation. Additionally, cells transfected with a DNA sequence encoding the Gab2 protein may be administered to a patient to provide an *in vivo* source of those proteins. For example, cells may be transfected with a vector containing a nucleic acid sequence encoding the Gab2 protein. The transfected cells may be cells derived from the patient's normal tissue, the patient's diseased tissue, or may be non-patient cells.

For example, tumor cells removed from a patient can be transfected with a vector capable of expressing the agent of the present invention, and re-introduced into the patient. The transfected tumor cells produce levels of the agent in the patient that inhibit Gab2 function, thereby, inhibit the growth of the tumor. Patients may be human or non-human animals. Cells may also be transfected by non-vector, or physical or chemical methods known in the art such as electroporation, ionoporation, or via a "gene gun." Additionally, the DNA may be directly injected, without the aid of a carrier, into a patient. In particular, the DNA may be injected into skin, muscle or blood.

The gene therapy protocol for transfecting the Gab2 agent into a patient may either be through integration of the Gab2 DNA into the genome of.the cells, into minichromosomes or as a separate replicating or non-replicating DNA construct in the cytoplasm or nucleoplasm of the cell. Expression of the GAb2 protein may continue for a long-period of time or may be reinjected periodically to maintain a desired level of the protein(s) in the cell, the tissue or organ or a determined blood level.

In one embodiment, the Gab2 peptides of the present invention can be used to raise antibodies against, e.g., specific for, Gab2. Such peptides can be used to immunize or vaccinate an animal. Thus, the invention encompasses antibodies and antisera, which can be used for testing of novel GAb2 proteins, and can also be used in diagnosis, prognosis, prevention or treatment of diseases and conditions characterized by, or associated with, Gab2 activity or lack thereof. Such antibodies and antisera can also be used to up-regulate or down-regulate Gab2 where desired.

Such antibodies and antisera can be combined with pharmaceutically-acceptable compositions and carriers to form diagnostic, prognostic or therapeutic compositions. The term "antibody" or "antibody molecule" refers to a population of immunoglobulin molecules and/or immunologically active portions of immunoglobulin molecules, *i.e.,* molecules that contain an antibody combining site or paratope.

Passive antibody therapy using antibodies that specifically bind the Gab2 protein can be employed to modulate Gab2-mediated, e.g., Gab2-dependent, processes such as degranulation, cytokine gene expression, and MAPK activation. In addition, antisera directed to the Fab regions of antibodies of the Gab2 protein can be administered to block the ability of endogenous antisera to the proteins to bind the proteins.

The antibodies of the present invention can be either polyclonal antibodies or monoclonal antibodies. These antibodies that specifically bind to the Gab2 proteins or with a protein that associates with Gab2 can be used in diagnostic methods and kits that are well known to those of ordinary skill in the art to detect or quantify the Gab2 proteins in a body fluid or tissue. Results from these tests can be used to diagnose or predict the occurrence or recurrence of a cancer and other Gab2-mediated diseases.

The invention also includes use of the Gab2 protein, antibodies to this protein, and compositions comprising this protein and/or its antibodies in diagnosis or prognosis of diseases characterized by Gab2 activity. As used herein, the term "prognostic method" means a method that enables a prediction regarding the progression of a disease of a human or animal, e.g., a mammal, diagnosed with the disease, in particular, an Gab2-dependent disease. The term "diagnostic method" as used herein means a method that enables a determination of the presence or type of Gab2-dependent disease in or on a human or animal.

The Gab2 protein can be used in a diagnostic method and kit to detect and quantify antibodies capable of binding the protein. These kits would permit detection of circulating antibodies to the Gab2 protein. Patients that have such circulating anti-Gab2 protein antibodies may be more likely to have Gab2-related disorders, such as cancers, immune disorders or allergic disorders, and may be more likely to have recurrences of cancer after treatments or periods of remission.

In one embodiment, the Fab fragments of these anti-Gab2 protein antibodies may be used as antigens to generate anti-Gab2 protein Fab-fragment antisera which can be used to neutralize anti-Gab2 protein antibodies to treat certain disorders caused by a decrease in Gab2 activity, e.g., that are caused by Gab2 underexpression, for example, immune disorder. Such a method would reduce the removal of circulating protein by anti-Gab2 protein antibodies.

The present invention is further illustrated by the following examples, which are not intended to be limiting in any way.

### EXAMPLES

### Example 1: Purification and sequencing of Gab2.

### Cell culture

Cell lines were grown in RPMI/10% FCS and the appropriate cytokine/growth factors.

### Northern blotting

Blots containing mouse tissue poly(A) RNA (Clontech, Palo Alto, CA) or total RNA (10 µg) from murine hematopoietic cells (provided by Dr. D. Zhang, Beth Israel Deaconess Medical Center, Boston, MA) were hybridized to radiolabelled cDNA probes, as indicated.

### Purification and Sequencing

Gab2 was purified from ~5 X 10¹⁰ P210BCR-ABL BaF3 cells. Affinity-purified rabbit anti-SHP-2 antibodies (2.6 mg) were crosslinked onto protein A Sepharose beads (Harlow, E. and Lane, D., Antibodies: A Laboratory Manual (Cold Spring Harbor, New York: Cold Spring Harbor Laboratory) (1988)) using dimethylpimelimidate (Pierce, Rockford, IL). P210 BCR-ABL BaF3 cells (4 x 10⁹) were resuspended in 40 ml hypotonic buffer (HB) containing protease and phosphatase inhibitors (Timms, J. F. et al., Mol. Cell. Biol.*18*, 3838-3850 (1998)) and homogenized. Lysates clarified at 100,000 x g were loaded onto Q-Sepharose and washed successively with HB and 20 mM Tris, pH 7.4/100 mM NaCI. Bound material was eluted in 20 mM Tris, pH 7.4/350 mM NaCl, diluted to a solution of 20 mM Tris7.4 /150 mM NaCl /0.2% NP40, incubated with the anti-SHP-2 antibody beads, and washed with five volumes each of 20 mM Tris, pH 7.4/500 mM NaCl and 100 mM glycine, pH 6.0. Bound material was eluted in 100 mM glycine, pH 2.5, and neutralized with 1M Tris, pH 8.0. Pooled eluates from 10 cycles of this protocol were concentrated (Centricon-30; Amicon, Bedford, MA), acetone-precipitated, and resolved by SDS-PAGE.

A fraction of the final preparation was transferred to a nylon membrane and stained for total protein (Amersham, Piscataway, NJ) and with anti-phosphotyrosine antibodies (anti-pTyr), and the immunoblots were examined. In addition to SHP-2 and Gab2, co-purifying species included an 85 kDa band, which was identified as the p85 subunit of PI-3K, and a band at ~150 kDa (p150). The 150 kDa species associated with SHP-2 only in some BCR-ABL-transformed hematopoietic cell lines (Gu, H. et al., J. Biol. Chem. *272,* 16421-16430 (1997)). Although SHP-2 was reported to associate with P210BCR-ABL (Tauchi, T. et al., J. Biol. Chem. *269*, 15381-7 (1994)), a ~210 kDa species was barely detectable in the final preparation, indicating that it is an association of low stoichiometry.

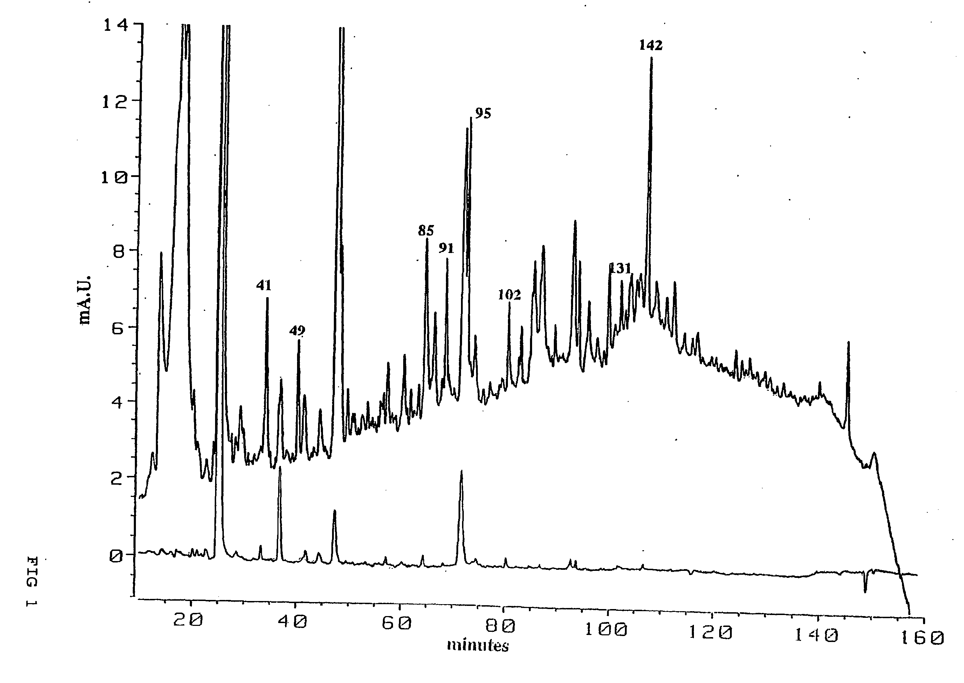

The 97 kDa band (which represented Gab2) was digested with endoprotease Lys-C. The resultant peptides were resolved by reverse-phase HPLC. The HPLC-resolved Lys-C peptides (nine peaks) were sequenced by Edman degradation (Figure 1), with additional support from MS/MS sequencing, by the Harvard Microchemistry Facility. These peptides (Table 1) did not match any protein in the database, indicating that the 97 kDa band was novel.

| Table 1. List of peptides obtained by Edman sequencing | | |
|---|---|---|
| Peptide No. | Sequence | Location in p97 |
| GK102 | (S/G/A) [G][G]* | 2-4 |
| GK124 | [Q]LEED[Y][Y][G][L][S](K)(G) | not present |
| PK85 | TQALQN[T]-(Q)* | 641-649 |
| PK91 . | [D][S]TYDLPR[S]LA* | 260-270 |
| GK41 | (Q/E/P)(I/S)(L/R)(D/H)(N/K)TEFK | 251-259 |
| GK49 | VD=VQVDK | 631-638 |
| GT131 | ELQDSFVFDIK | 81-91 |
| GT142 | AKPTPLDLRNNTVIDEL* | 512-529 |
| GK95 | SSLTGSETDNEDVYTFK | 277-293 |

| | | |
|---|---|---|
| Note: [ ]: Amino acids were determined with reasonable probability. | | |
| ( ): Amino acids were determined with low probability. The amino acids which are present in the Gab2 protein are underlined. *: these peptides are incomplete sequences. | | |

### Example 2: Cloning of Gab2

Reverse transcription-polymerase chain reaction (RT-PCR) was used to obtain a cDNA fragment corresponding to peptide GT142, and this fragment was used to clone full length Gab2 cDNAs. Degenerate primers corresponding to all possible codons for the sequences KAKPTP (SEQ ID NO: 1) and TVIDEL (SEQ ID NO: 2) in peptide GT-142 (Table 1) were synthesized and used in a RT-PCR reaction with total RNA from P210BCR-ABL BaF3 cells. The expected 68 bp PCR product was subcloned into pUC19. Three inserts were sequenced and found to encode GT-142. A unique sequence (5'CCTTGACCTGAGAAACAACAC3') (SEQ ID NO: 3) encoding the middle of GT-142 (LDLRNN)(SEQ ID NO: 4) served as the 5' primer in a 3'RACE reaction (GIBCO-BRL, Rockville, MD), yielding a single 800 bp product. Its sequence revealed a single open reading frame containing peptides GT142, GK49 and PK85 (Figure 2; Table 1). The 800 bp product was used as to probe a BaF3 cell cDNA library in λUniZap (provided by Dr. Alan D'Andrea, Dana Farber Cancer Institute, Boston, MA). Twenty positive clones were obtained, the two largest containing ~5 kb inserts. Plasmids containing cDNAs were recovered by superinfection, according to the manufacturer's instructions (Stratagene, La Jolla, CA).

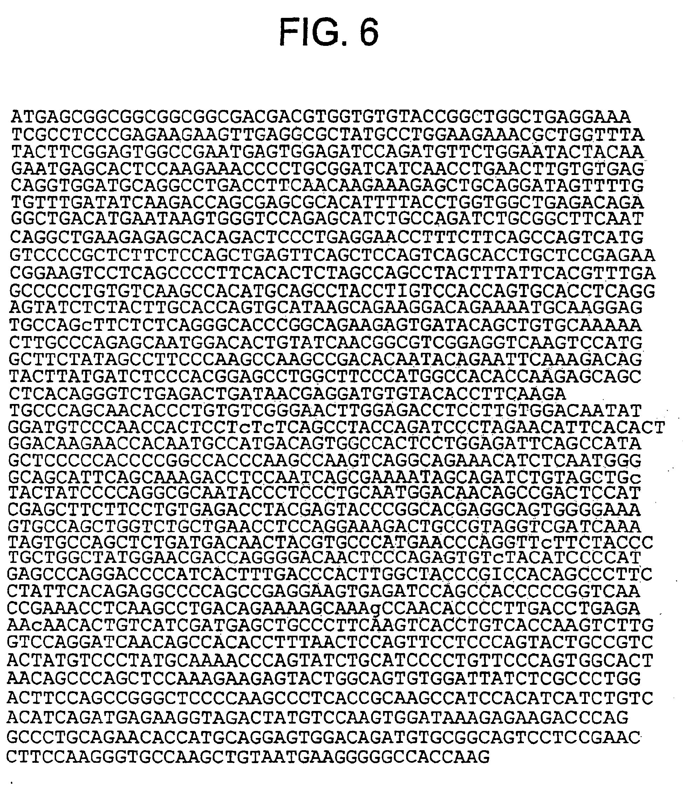

The two largest clones contained the consensus Kozak sequence specifying transnational initiation (GAC ATG AGC), an in-frame upstream stop codon, and a single long (1998 bp) open reading frame. The sequence predicted a 666 amino acid protein with a calculated molecular weight of 73 kDa (Figure 2). The nucleotide sequence is deposited as GenBank Accession Number AF104244 (Figure 6). Eight of nine Lys-C peptides obtained by microsequencing were found within the predicted sequence (Figure 2 and Table 1), strongly suggesting that this cDNA encodes Gab2. Presumably, the one missing peptide derived from a trace contaminant.

This cDNA must encode *bona fide* Gab2 from BaF3 cells, because peptide GK102, comprising the p97 N-terminus, is predicted by this cDNA sequence, although it is absent in the sequence of unknown gene KIAA0571 (Figure 3 and Table 1). KIAA0571 was deposited in GenBank (Accession Number AB011143) as part of a collection of human brain cDNAs (Nagase et al., DNA Res. *5*, 31-39 (1998)). The KIAA0571 cDNA encoded a protein with 88% amino acid identity to Gab2, suggesting that KIAA0571 may be the human homolog of Gab2. The nucleotide sequences of the 5' ends of Gab2 and KIAA0571 are highly divergent, such that the first 39 amino acids of Gab2, which comprises part of the PH domain, were absent in KIAA0571. In the absence of this sequence information, the likely function(s) of Gab2 cannot be deduced. In addition, the function of KIAA0571 open reading frame, were it to be expressed in vivo, is almost certain to be compromised, since PH domains bind to phospholipids and promote targeting to cellular membranes. KIAA0571 may represent an alternatively spliced form of Gab2, although cloning artifacts in KIAA0571 cannot be excluded.

The Gab2 protein contained an N-terminal PH domain that has a potential Grb2 SH2 domain binding site, followed by a long region with multiple potential tyrosyl phosphorylation sites, two serine-rich stretches (a.a. 126-188 and 532-600, respectively), and 2 PXXP sites, potentially capable of binding SH3 or WW domains (Figures 2, 3 and Table 2). Mammalian Gab1 and *Drosophila* Dos have similar topography and some sequence similarity to Gab2 (Figures 3, 4). The greatest similarity resides in their PH domains, with 73% identity between Gab2 and Gab1, and 45% identity between Gab2 and Dos (Figure 4; top panel). Most of the potential phosphorylation sites and their relative positions within each protein are conserved, as is one of the PXXP motifs (Figure 3 and Table 2).

| Table 2. List of potential SH2 domain binding motifs in Gab2 | |
|---|---|
| Potential Tyrosyl Phosphorylation Sites | SH2 Domain Containing proteins |
| YKNE | Grb2 |
| YSLP | PLCγ |
| YDLP | Crk or Nck |
| YQIP | Crk or PLCγ |
| YEYP | Crk |
| YVPM | p85 (PI3-K) |
| YIPM | p85 (PI3-K) |
| YVPM | p85 (pI3-K) |
| YLAL | SHP-2 |
| YVOV | SHP-2 |

There are also potentially significant sequence differences between these proteins. The Met binding domain (MBD) of Gab1 (a.a.450-532) mediates association with c-Met/HGFR (Weidner et al., Nature 384, 173-176 (1996)). The cognate region in Gab2 (a.a. 443-514) exhibits only 36% amino acid identity (Figure 4, bottom panel). The MBD, of Gab1, but not Gab2, contains two proline-rich stretches that comprise potential Grb2 SH3 domain-binding sites (Yu, H. et al., Cell *76*, 933-945 (1994)). Conversely, two potential 14-3-3 binding sites (Yaffe, M. B. et al., Cell *91*, 961-971 (1997)) are present only in Gab2 (RKS₁₆₀SAP, and RQS₆₅₈SEP). It remains to be determined whether Gab2 binds 14-3-3 proteins *in vivo.*

### Example 3: Confirmation of Gab2 cDNA

To confirm that the cDNA encoded Gab2, a vector directing expression of HA-tagged GAb2 construct (Gab2HA) was transiently transfected into BaF3 cells. BaF3 cells were washed in serum-free RPMI, resuspended at 10⁷ cells/0.5 ml in RPMI/10% FCS, and incubated (10 minutes) with the indicated amounts of Gab2 expression vector and/or 20 µg of the SHP2 expression vector, the indicated amount of promoter luciferase reporter, and 20 ng of Renilla luciferase-TK reporter (Promega, Madison, WI).

Constructs encoding Gab2 (Gab2HA), Gab2 lacking amino acids 604-662 (Gab2ΔY2HA), and Gab2 with tyrosines 604/633 mutated to phenylalanine (Gab2DM), all with C-terminal HA tags, were constructed by PCR, using Gab2 cDNA as the template. PCR products were cloned into pEBB (from Dr. B. Mayer, Children's Hospital, Boston, MA), which directed expression under the control of the elongation factor 1-α promoter. The transfected Gab2HA fusion protein, as well as endogenous Gab2, migrated at an apparent mobility of about 97 kDa. Upon IL-3 stimulation, Gab2HA became tyrosyl phosphorylated and co-immunoprecipitated with SHP-2, consistent with its expected properties (Gu, H. et al., J. Biol. Chem. *272*, 16421-16430 (1997)).

Further evidence was provided by studies with specific antibodies. A fragment encoding Gab2 a.a. 523-666 was subcloned in frame into pGEX 4T-1 (Pharmacia, Piscataway, NJ). GST fusion protein was produced as described (Lechleider et al., J. Biol. Chem. 268, 13434-13438 (1993)). Affinity purified antibodies were prepared by passing antisera sequentially over GST and GST-Gab2 bound to Affi-Gel 15 (Bio-Rad, Hercules, CA), prepared as described (Frangioni et al., Cell 68, 545-560 (1992)). 4 µg of affinity purified antibodies quantitatively deplete Gab2 from 10' BaF3 cells. Rabbit antibodies against peptide GT142 coupled to KLH were generated by BABCO (Berkeley, CA).

### Immunoprecipitations and immunoblotting.

Cell lysis, immunoprecipitation, immunoblotting, and detection by enhanced chemi-luminescence (Amersham, Piscataway, NJ) were performed (Timms et al., Mol. Cell. Biol. *18*, 3838-3850 (1998)). Monoclonal antibody 9E10 (against the myc-epitope) was obtained from BABCO (Berkeley, CA). Monoclonal anti-phosphotyrosine antibody 4G10 was obtained from UBI (Santa Cruz). Anti-SHP2 immunoprecipitations utilized 1µg antibody/10⁷ BaF3 cell equivalents. Anti-Gab2 immunoprecipitations utilized 4µg antibody/10⁷ BaF3 cell equivalents. Dilutions for immunoblotting were: anti-SHP-2 (1:2500); anti-Grb-2 (1:1000, Santa Cruz), anti-Shc (1:1,000, Transduction Laboratories, Lexington, KY); anti-p85 (1:3500, from Dr. C. Carpenter, Beth Israel Deaconess Medical Center); anti-MAPK (1:5,000, from Dr. J. Blenis, Harvard Medical School, Boston, MA); anti-Gab2 peptide antibodies (1:500); anti-GST-Gab2 antibodies (1:2500) and anti-pTyr antibodies (0.5 µg/ml).

BaF3 cells, starved in RPMI/0.8% BSA for 6 hours, were stimulated with recombinant IL-3 (10 ng/ml). Polyclonal antibodies against peptide GT-142 (Table 1) detected a 97 kDa protein in SHP-2 immunoprecipitates from IL-3-stimulated BaF3 cells; this protein co-migrated with the 97 kDa phosphotyrosyl protein associated with SHP-2. Antibodies against GST-Gab2 specifically immunoprecipitated 97 kDa and 70 kDa tyrosyl phosphorylated proteins from IL-3-stimulated BaF3 cells; these proteins co-migrated with the tyrosyl phosphoproteins present in anti-SHP-2 immunoprecipitates. Probing these immunoblots with anti-GST-p97 antibodies confirmed that the 97 kDa protein immunoprecipitated with anti-Gab2 and anti-SHP-2 antibodies is Gab2.

These data established that Gab2 is a 97 kDa SHP-2 binding protein. To determine whether Gab2 is the only component of the 97 kDa tyrosyl phosphorylated band associated with SHP-2 in IL-3-stimulated BaF3 cells, immunodepletion studies were performed. Quantitative depletion of Gab2 left no remaining 97 kDa phosphotyrosyl protein(s) in SHP-2 immunoprecipitates. Likewise, nearly all of the Gab2 in BaF3 cells associated with SHP-2 upon IL-3 stimulation (as indicated by a comparison of intensities of 97kDa band in anti-Gab2 and anti-SHP-2 lanes), suggesting that SHP-2 probably is critical for signals emanating from Gab2. In contrast, only ~10% of SHP-2 was found in a complex with Gab2 upon IL-3 stimulation. This excess of SHP-2 is consistent with the possibility that it interacts with additional targets in BaF3 cells.

### Example 4: Gab2 Expression and Response to Diverse Hematopoietic Stimuli

Since a 97 kDa protein was only observed associated with SHP-2 in hematopoietic cells, Gab2 was expected to be hematopoietic cell-specific. Indeed, Gab2 was expressed in many (but not all) hematopoietic cell lines, representing multiple lineages, and was not expressed in fibroblasts. Surprisingly, however, a 6 kb Gab2 transcript was observed in most tissues, with two additional smaller transcripts found in testis. Expression of Gab2 was highest in heart, testis, and lung, with lower levels in brain and liver. Although Gab2 participates in lymphocyte signaling, its expression was relatively low in spleen and thymus. Gab1 expression in the same tissues was largely overlapping, suggesting that Gab1 and Gab2 are co-expressed in at least some cell types. The relative levels of Gab2 and Gab1 expression are not the same in all tissues (as indicated by a comparison expression in brain, liver, and testis).

SHP-2 associated with proteins of similar size to Gab2 (95-110 kDa) in many signaling cascades, so Gab2 tyrosyl phosphorylation in response to a range of stimuli was examined. Kit225 cells (from Dr. P. Brennan, ICRF, London, U.K.), starved in RPMI/10% FCS for 48 hours, were stimulated with IL-2 (25 units/ml). BAC1.2F5 cells were stimulated as described (Timms et al., Mol. Cell. Biol. *18*, 3838-3850 (1998)). WEHI 231 cells were stimulated with 15 µg/ml goat F(ab)'₂ anti-mouse IgG (Jackson ImmunoResearch Laboratories, West Grove, PA). Jurkat cells were stimulated with 1 µg/ml anti-CD3 antibody, OKT3 (ATCC), and crosslinked with 10 µg/ml rabbit anti-mouse IgG.

SHP-2 associated with a 100 kDa p-Tyr protein upon CSF-1 stimulation of myeloid progenitor cell lines (Carlberg and Rohrschneider, J. Biol. Chem. *272*, 15943-15940 (1997)) or macrophages (Timms et al., Mol. Cell. Biol. *18*, 3838-3850 (1998)). CSF-1 stimulation of BAC1.2F5 cells resulted in the rapid tyrosyl phosphorylation of Gab2 and its association with SHP-2. Similar results were obtained following IL-2 stimulation of Kit 225 cells or erythropoietin stimulation of erythropoietin receptor-expressing BaF3 cells. In Jurkat cells, TCR stimulation results in association of SHP-2 with a "110 kDa" p-Tyr protein (Frearson et al., Eur. J. Immunol. *26*, 1539-1543 (1996); Frearson and Alexander, J. Exp. Med. *187*, 1417-1426 (1998)); in these cells, Gab2 was rapidly tyrosyl phosphorylated and associated with SHP-2. B cell receptor (BCR) stimulation of WEHI-231 cells also led to Gab2 phosphorylation.

### Example 5: Association of Gab2 with other Signaling Molecules

The ability of Gab2 to also associate with other signaling molecules was examined. Tyrosyl phosphorylation of Gab2HA occurred within 1 minute of IL-3 stimulation of BaF3 cells, and then increased further, peaking by 10 minutes and accompanied by a dramatic decrease in Gab2 electrophoretic mobility. Besides SHP-2, tyrosyl phosphorylated Gab2 associated with Shc and the p85 subunit of PI-3K. In contrast, consistent with its PXXP motifs, Gab2 associated constitutively with Grb2. Shc, p85 and Grb2 also co-immunoprecipitated with endogenous Gab2 from IL-3-stimulated BaF3 cells. Sites within Gab2 conform to the consensus for the SH2 domains of Crk (and Crk-II and Crk-L) and PLCγ (Figure 3 and Table 2), but using available immunoreagents, endogenous Gab2 or Gab2HA association with these proteins was not detected. Although Grb2 binds Gab2, Sos was not detected in Gab2 immunoprecipitates, perhaps because Grb2 binds primarily via its SH3 domain to Gab2.

### Example 6: Role of Gab2 and the Gab2/SHP-2 interaction in IL-3 signaling

The effects of expressing wild type Gab2 and Gab2 mutants unable to bind SHP-2 on IL-3 signaling were examined. Two tyrosines within Gab2 (Y₆₀₄LAL/ Y₆₃₃VQV) conform to the consensus for binding the SH2 domains of SHP-2 (Songyang et al., Cell *72*, 767-778 (1993)); both sites are highly phosphorylated. A deletion mutant lacking these residues (a. a. 604-666) was generated, and a C-terminal HA tag was appended, as described in Example 5. This mutant (Gab2ΔY2HA) or wild type Gab2HA was transiently transfected into BaF3 cells.

Although Gab2ΔY2HA was expressed, it did not bind SHP-2 upon IL-3 stimulation. IL-3-induced tyrosyl phosphorylation of Gab2ΔY2HA was similar, or even slightly less than that of wild type Gab2HA. The minimal effect on Gab2 tyrosyl phosphorylation observed upon mutating its SHP-2 binding sites contrasts with its increased tyrosyl phosphorylation upon over-expression of catalytically inactive mutants of SHP-2. Although C-terminal deletion eliminated SHP-2 binding, Gab2 association with p85 and Shc was not decreased, suggesting that Gab2 structure was not grossly perturbed in Gab2ΔY2HA. Grb2 binding to Gab2ΔY2HA also decreased slightly, particularly upon IL-3 stimulation, most likely because some Grb2 associates indirectly with Gab2 via binding to tyrosyl phosphorylated SHP-2 (Welham et al., J. Biol. Chem. *269*, 23764-23768 (1994)).

SHP-2 is required for induction of c-fos. Therefore, the role of Gab2 in IL-3-induced c-fos promoter activity was assessed. For c-fos reporter assays, 1.5 µg of c-fos promoter (nt -710 to +42)-luciferase reporter were used (Hu et al., Science 268, 100-102 (1995)). STAT-driven transctivation was measured using 1.5 µg of a GAS-luciferase reporter (Jaster et al., Mol. Cell. Biol. 3364-3372 (1997)). For Elk assays, Gal4-Elk-1 (2 µg) and Gal4-luciferase (1 µg) were used (Bennett et al., Mol. Cell. Biol. 16, 1189-1202 (1996)). Cells were electroporated at 300V/800µF, transferred to fresh RPMI/10% WEHI supematant/10% FCS and, after three hours, were starved in RPMI /10% FCS. Twelve hours post-transfection, transfected cells (approximately 10⁶/condition) were incubated in RPMI/10% FCS alone or with murine IL-3 (1 ng/ml) for two hours. Luciferase assays were performed with a kit (Promega, Madison, WI). Promoter activities were normalized to Renilla luciferase levels.

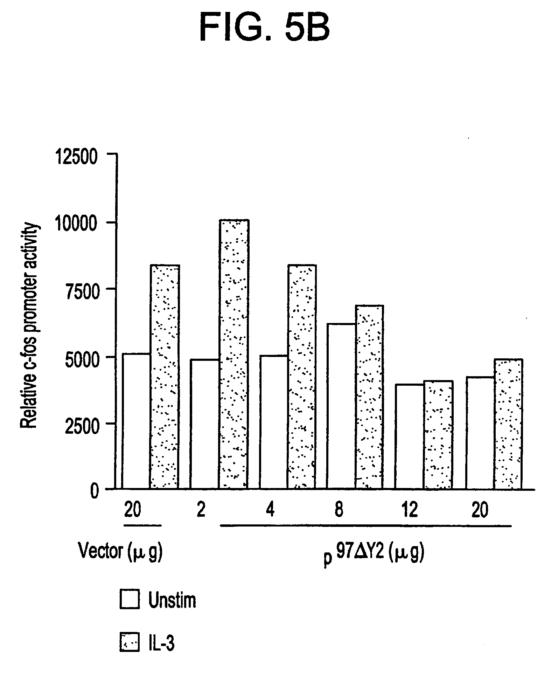

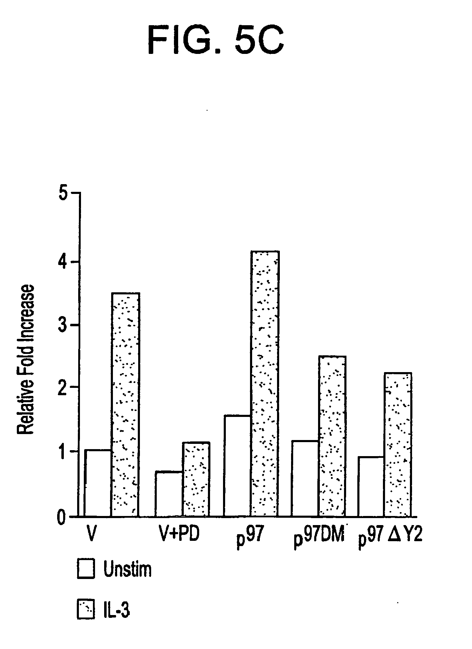

Over-expression of wild type Gab2HA had little effect on IL-3-stimulated c-fos luciferase activity, although it did evoke a small (~2-fold), but reproducible, increase in basal activity (Figure 5A). However, expression of a comparable level of Gab2ΔY2HA decreased IL-3-evoked c-fos reporter activity (Figure 5A) in a dose-dependent manner (Figure 5B). Presumably, Gab2ΔY2HA displaced endogenous Gab2 from its proper location *in vivo* but, unable to bind SHP-2, Gab2ΔY2HA cannot transmit a signal necessary for full activation of c-fos. Dominant negative SHP-2 (SHP2ΔP) inhibited IL-3 induced c-fos activation (Figure 5A), and the increased basal c-fos luciferase activity evoked by Gab2HA was blocked by dominant negative SHP-2. A Gab2 mutant in which Y604 and Y633 were converted to phenylalanine (Gab2DM) also lacked SHP-2 binding and inhibited IL-3-evoked c-fos luciferase activity (Figure 5C). These findings suggest that Gab2 function, and, in particular, Gab2 binding to SHP-2, are required for full cytokine-induced c-fos activation.

The SRE (serum response element), which binds the SRF/TCF (serum response factor/TCF) complex is required for c-fos activation. MAPK phosphorylates Ets family transcription factors that comprise TCF, (e.g., Elk-1), increasing their transactivation potential. The ability of a Gal4 -Elk-1 fusion to drive GAL4-luciferase activity in response to IL-3 was inhibited by Gab2ΔY2HA, indicating Gab2/SHP-2 was required for Elk-driven transactivation. STAT 5 also contributed to activation of the c-fos promoter in response to IL-3/GM-CSF (Rajotte et al., Blood *88*, 2906-2916 (1996)). The Gab2/SHP-2 complex was required for full activation of a STAT-responsive element as well. Thus, Gab2/SHP-2 association was required for full cytokine-induced activation of the two major elements in the c-fos promoter.

The MEK inhibitor PD98059 ablated IL-3-induced c-fos luciferase activity, indicating that MEK/MAPK activation is essential for IL-3-induced c-fos promoter activation in BaF3 cells. Gab2ΔY2HA was expected to also inhibit IL-3-induced MAPK activity, particularly since SHP-2 is required for cytokine-induced MAPK activation. Transient over-expression of wild type Gab2HA potentiated MAPK activation in response to IL-3, suggesting that Gab2 can signal to MAPK. Surprisingly, however, Gab2ΔY2HA or Gab2DM not only failed to inhibit, but potentiated IL-3-evoked MAPK activation.

Because of these surprising findings, the role of SHP-2 in IL-3-induced MAPK activation was re-examined. For MAPK assays, BaF3 cells (10⁷) were co-transfected with 20 µg Gab2 or SHP2 expression plasmids and 2 µg of myc-tagged Erk1. Cells (5 x10⁶) were stimulated with murine IL-3 for various times, washed, lysed in NP40 buffer, and Myc-Erk was immunoprecipitated with 9E10 (1 µl of ascites/sample). Kinase assays using myelin basic protein (MBP) were performed as described (Bennett et al., Mol. Cell. Biol. 16, 1189-1202 (1996)).

As mentioned above, previous work showed that SHP-2 is necessary for IL-3-induced MAPK activity. Consistent with these reports, two mutants of SHP-2, SHP2ΔP and SHP2CS inhibited IL-3-induced MAPK activation. It was concluded that the Gab2/SHP-2 complex was required for full activity of the c-fos promoter, acting on Ets and STAT family transcription factors via a cascade parallel to MAPK activation, and that SHP-2 must act at more than one point in cytokine signaling.

### Example 7: Characteristics of Gab2-/- BMMCs

Wild type (WT) and Gab2 knockout (-/-) BMMCs were incubated with 2 ug/ml anti-DNP monoclonal IgE (SPE-7) for 1 hour on ice. Cells were washed and incubated with FITC-anti-IgE rat monoclonal antibody for 30 minutes. Cell surface expression of FcεRI were determined by FACS, and found to be normal in Gab2-/- BMMCs.

BMMCs were sensitized with anti-DNP IgE, and stimulated with 10 ng/ml anti-dinitrophenol (DNP). Cells were lysed, and total cell lysates were resolved by SDS-PAGE, western blotted with anti-phosphotyrosine antibody (pTyr), and reprobed with anti-Akt antibodies. Total tyrosyl phosphorylation was found to be normal in Gab2-/BMMCs upon FcεRI engagement.

BMMCs were sensitized with anti-DNP-IgE, stimulated with 10 ng/ml DNP, and lysed. Syk and LAT were immunoprecipitated by anti-Syk and LAT antibodies, resolved by SDS-PAGE, and western blotted with anti-phosphotyrosine antibody (pTyr), and reprobed with Syk antibodies. Syk and LAT tyrosyl phosphorylation are normal in Gab2-/- BMMCs upon FcεRI engagement.

BMMCs were sensitized with DNP mouse IgE (2 ug/ml) for 12 hours and labeled with ³H-serotonin for 3 hours. Unincorporated label was washed away and cells were stimulated for 15 minutes with the indicated concentrations of DNP. Serotonin released into the media and remaining in the cell pellet was quantified by scintillation counting, and it was determined that FcεRI-mediated degranulation is impaired in Gab2-/- BMMCs.

BMMCs were sensitized with anti-DNP-IgE, and stimulated with 10 ng/ml DNP for 0 and 1 hour. Total RNAs were isolated from BMMCs, and reverse-transcribed into first cDNA by reverse-transcriptase. The relative level of TNFα and IL-6 cDNAs in each sample was determined by Real time PCR, and Fcε-evoked TNFα and IL-6 gene expression are impaired in Gab2-/- BMMCs.

BMMCs were sensitized with anti-DNP-IgE, stimulated with 10 ng/ml DNP, and lysed. PLCγ1 was immunoprecipitated by anti-PLCγ1 antibodies, resolved by SDS-PAGE, and western blotted with anti-phosphotyrosine antibody (pTyr), and reprobed with anti-PLCγ1 antibodies. It was determined that tyrosyl phosphorylation of PLCγ is impaired in Gab2-/- BMMCs upon FcsRI crosslinking.

BMMCs were sensitized with anti-DNP IgE, and stimulated with 10 ng/ml DNP. Cells were lysed, and total cell lysates were resolved by SDS-PAGE, western blotted with anti-phospho-Akt antibodies, and reprobed with anti-Akt antibodies. Akt phosphorylation was found to be impaired in Gab2-/- mast cells upon FcεRI engagement.

BMMCs were sensitized with anti-DNP IgE, and stimulated with 10 ng/ml DNP. Cells were lysed, and total cell lysates were resolved by SDS-PAGE, western blotted with anti-phospho-Akt, phospho-p38, and phospho-MAPK antibodies, and reprobed with anti-Akt and MAPK antibodies. JNK and p38 phosphorylation is defective in Gab2-/-BMMCs, however, MAPK phosphorylation is not affected in Gab2-/-BMMCs.

Gab2-/-BMMCs were infected with MSCV-IRES-GFP virus expressing wild type Gab2 (WT) or virus alone (Vector). GFP positive BMMCs were sorted out by FACs, expanded, sensitized by IgE, and stimulated with 10 ng/ml DNP-HSA. Cells were lysed, and equal amount of total cell lysates were resolved by SDS-PAGE, transferred, and blotted with anti-phospho-Akt (473), phopho-JNK, phospho-38, and phospho-MAPK respectively. The same blot was reprobed with anti-Akt and Erk2. Expression of wild type Gab2 rescues signaling defects in Gab2-/-BMMCs.

20 ng of anti-DNP-IgE was injected intradermally into one ear of the mice. 24 hours later, 100 µg DNP in 200 µl of PBS with 2% Evans Blue was injected into the tail vein of the mice. 30 minutes later, the mice were sacrificed, and the ears were removed, cut into small pieces. Evans blue dye was extracted from the ears with formamide and 80°C incubation for 3 hours, and quantified by reading OD at 610nm. Passive cutaneous anaphylaxis was found to be defective in Gab2-/- mice.

### Example 8: Location of Gab2 gene on human chromosome 11q13.3-14.2

Gab2 was purified and cloned from BCR-ABL transformed cells (Gu, H. et al., Mol Cell. 2, 729-740 (1998)). Unpublished data indicated that Gab2 can be phosphorylated by BCR-ABL *in vitro.* To further explore potential Gab2 involvement in human disease, FISH analysis was performed to localize the Gab2 gene on human genome. The Gab2 gene was located on human chromosome 11q13.3-14. Importantly, chromosome 11q13 is amplified in about 15% of primary breast cancers (Hui, R. *et al.,* Oncogene 15, 1617-1623 (1997)). Cyclin D1, also located in the 11q13 amplicon, is one of the few genes expressing in the breast tumor bearing this amplicon (Siegel, P. et al., Bioessays 22, 554-563 (2000)). The Gab2 gene may be amplified and expressed in breast tumors with the 11q13 amplicon.

### Example 9: Overexpression of Gab2 protein in breast cancer cell lines and breast tumor cells

The expression of Gab2 in breast cancer cell lines and breast tumor samples was examined by western blot analysis. The Gab2 protein was overexpressed in ~40% breast cancer cell lines (16 total) examined, such as MDA-MB-134, 468, BT-20, T47D, MDA-MB-435, 21NT. In contrast, Gab2 protein is just above detectable level in immortalized normal human mammary epithelia cell lines MCF-10A and 184B5. Furthermore, Gab2 protein level was high in ~20% breast tumor samples (total 30 samples) compared to normal breast tissue.

In collaboration with Dr. Qian Wu at Brown University Hospital in Rhode Island, Gab2 expression in five breast tumor samples was examined by immunohistochemistry using anti-Gab2. Importantly, there was strong Gab2 immunostaining in breast carcinoma cells of all the examined samples. Strong Gab2 inununostaining was found in carcinoma cells from both invasive ductal carcinoma and DCIS tumor samples. In contrast, the normal mammary epithelial ducts as well as the surrounding normal connective tissues showed weak Gab2 immunostaining, confirming that Gab2 is overexpressed specifically in tumor cells.

### Example 10: EGF induction of Gab2 tyrosyl phosphorylation in breast cancer cell line

Since overexpression of EGFR family members (ErbB2) can promote breast carcinogenesis, the overexpressed Gab2 may be involved in breast cancer by amplifying EGFR initiated signals. Gab2 tyrosyl phosphorylation and its association with signal relay molecules by Gab2 immunoprecipitation followed by immunoblotting with p85 and SHP-2 antibodies was examined. Gab2 was found to be robustly tyrosyl phosphorylated and became associated with p85 and SHP-2 upon EGF stimulation in MDA-MB-486 cells.

### Example 11: Generation of mammary epithelial cell lines overexpressing Gab2

Gab2 was overexpressed in two well-characterized breast epithelial cell lines to investigate the effects of Gab2 overexpression on breast cell growth. One breast epithelial cell line was the immortalized normal epithelial cell line MCF-10A. The other was the breast cancer cell line MCF-7, which expressed a low level of Gab2. MCF-7 clones that can inducibly express Gab2 using the tetracycline-off expression system were generated (Gossen, M. & Bujard, H., Proc Natl Acad Sci, USA 89, 5547-5551 (1992)). The advantage of using this expression system was that Gab2 expression level can be controlled depending on the different concentration of tetracycline present in the culture media. These Gab2 overexpressing cell lines will be useful tools to study the effect of Gab2 on breast cell growth *in vitro* and tumor formation in mice.

### Example 12: Effects of Gab2 overexpression on mammary epithelial cell lines

Breast cancers mainly arise from breast epithelia, and proceed through a series of changes starting with hyperplasia with atypia and progressing to *carcinoma in situ,* invasive carcinoma, and eventually metastatic disease. At different stages of the carcinogenesis, breast cancer cells become highly proliferative, resistant to apoptosis, grow without maintaining polarity and/or their dependence on basement membrane, and more migratory and invasive. Mammary epithelial cell lines with stable expression of Gab2 were established, whether Gab2 overexpression enhances or promotes growth, migration/invasion, and transformation of mammary epithelial cell lines, such as immortalized mammary epithelial cells (MCF-10A) and breast cancer cell (MCF-7), *in vitro* can be assessed. The effects of Gab2 overexpression on tumorigenicity in nude mice can also be assessed.

### Example 13: Effects of Gab2 overexpression on the inunortalized mammary epithelial cell line MCF-10A

MCF-10A cell growth mainly depends on the presence of EGF in the culture medium since EGF withdrawal causes MCF-10A cells to arrest at G0/G1 (Blagosklonny, M. V. et al., Cancer Res 60, 3425-3428 (2000)) and eventually become apoptotic (Davis, J. W. et al., Carcinogenesis 21, 881-886 (2000)). First, the issue of whether Gab2 overexpression promotes MCF-10A growth in its growth medium containing EGF can be examined. An equal number of MCF-10A vector-transfected cells (V) and two clones overexpressing Gab2 can be plated in growth media. Cells can be counted every day for three days by trypan-blue exclusion. If the Gab2 overexpressing cells grow faster in this assay, the issue of whether Gab2 expression increased MCF-10A proliferation and/or decreased cell death can be determined. To quantify dead cells, cell samples can be taken every 24 hours for three days and assayed for apoptotic cells using Annexin V (which binds to the cell surface of the early apoptotic cells) reagent.

The cell cycle length of MCF-10A-V and -Gab2 cell lines can be measured to examine whether Gab2 expression increases MCF-10A cell proliferation. Appropriate cells can be pulse labeled with BrdU. Then every 3 hours for 24 hours (the typical doubling time of MCF-10A cells), an aliquot of the labeled cells can be taken and the BrdU+ cells relative to DNA content can be analyzed by Propidium Iodide (PI). Initially, essentially all BrdU+ cells should be in S phase (by PI staining). Over time, the BrdU+ cells should move progressively from S phase into G2/M, G1, and back to S phase. The length of each cell cycle phase can be determined by calculating how long it takes BrdU+cells to traverse all the phases. A shorting of either G1, S or G2/M phase of MCF-10A-Gab2 cells compared to MCF-10A-V cells can indicate which phase of the cell cycle is affected by the Gab2 overexpression. Since EGF may induce Gab2 tyrosine phosphorylation and its association with p85 and SHP-2, but not insulin/IGF-1, Gab2 may promote MCF-10A cell growth (increase cell proliferation and/or survival) through the amplification of the EGF-initiated signal transduction.

Two assays can be applied to examine whether Gab2 overexpression causes MCF-10A growth independent of polarity and substratum *in vitro*. First, the ability of MCF-10A-Gab2 cells to undergo anchorage-independent growth can be tested. Equal number of MCF-10A-V and MCF-10A-Gab2 cells can be seeded in soft agar in growth media. Two weeks later, the number of colonies containing more than four cells can be scored as positive (Zelinski, D. P. et al., Cancer Res 61, 2301-2306 (2001)). Next, the issued of whether Gab2 overexpression can disrupt the formation of normal mammary tissue structures (acini) by the parental MCF-10A cells when grown on Matrigel, another property of breast cancer cells, can be tested (Weaver, V. M. et al., Semin Cancer Biol 6, 175-184 (1995)). Both MCF-10A-V and -Gab2 cells can be seeded on Matrigel coated dishes in growth media. 7-10 days later, the formation of acini (a spherical structure with a single layer of cells surrounding a hollow lumen) can be examined by confocal microscopy. If the formation of disorganized cell aggregates on Matrigel and/or the appearance of positive colonies in soft agar was observed in cells overexpressing Gab2, this could suggest that Gab2 overexpression was oncogenic to normal breast epithelial cells.

Another property of aggressive breast cancer cells is that they are migratory and invasive. The effect of Gab2 expression on migration and invasion of MCF-10A cells using transwell assay can be examined. For a migration assay, cells can be seeded in the upper chamber of the transwell containing semi-permeable filters. A time course (2, 4, 6, 8 hours after incubation ) of cells migrating to the bottom side of the filter can be determined. To measure invasion, a similar assay can be performed except the transwell filter can be coated with Matrigel. To measure the kinetics of cells invading through the matrix and getting onto the other side of the membrane, a longer time course of measurement can be followed.

Because *in vitro* transformation assays do not always predict tumorigenic potential *in vivo,* the issue of whether MCF-10A-Gab2 cells cause tumor formation when subcutaneously injected into nude mice can be examined. If MCF-10A-Gab2 cells are tumorigenic in nude mice, this would indicate that Gab2 overexpression may promote tumorigenesis *in vivo.*

Since PI-3K and SHP-2 are two major effectors of Gab2 (Gu, H. et al., Mol Cell. 2, 729-740 (1998); Gu, H. *et al.,* Nature 412, 186-190 (2001)), the issue of whether Gab2 activated PI-3K and SHP-2 or both contribute to the potential increased growth and transformation of MCF-10A cells can be investigated. To address this question, Gab2 mutants that cannot bind PI-3K (ΔPI3K), SHP-2 (ΔSHP2), and both PI-3K and SHP-2 (ΔPI3K+SHP2) in MCF-10A cells can be expressed using the pBabe-puro retroviral vector. Pools or multiple clones (at least 3 each) of MCF-10A cells expressing similar level of Gab2 wild type (WT) and Gab2 mutants can be used for the proliferation, apoptosis, transformation, and migration/invasion assays mentioned above. One of the Gab2 ΔPI3K, ΔSHP2 and double mutants would be expected to be defective in enhancing some of the MCF-10A responses. In case all of these Gab2 mutants behave in the same way as Gab2 WT, the effects of expressing other Gab2 mutants, such as Gab2 mutant that cannot bind Crk (Crouin, C. et al., FEBS Lett 495, 148-153 (2001)), can be tested on MCF-10A growth. It has been reported that Gab1 binding to Crk correlates to the ability of Gab 1 to promote transformation by oncogenic Met (Lamorte, L. et al., Oncogene 19, 5973-5981 (2000)).

### Example 14: Gab2 cooperation with ErbB2 on transformation of MCF-10A cells.

It is conceivable that Gab2 overexpression only enhances MCF-10A proliferation or survival, but not transformation or tumorigenicity of MCF-10A due to the limited number of ErbB1 (EGFR) and ErbB2 on the surface of MCF-10A cells. Overexpression of Gab2 alone may not be enough to activate oncogenic cascades in MCF-10A cells.

ErbB2 overexpression in mammary gland can induce breast tumor in mice (Guy, C. T. *et al*. Proc Natl Acad Sci U S A 89, 10578-10582 (1992)). Interestingly, overexpressing ErbB2 cannot transform MCF-10A cells and MCF-10A-ErbB2 cells are not tumorigenic when injected into mice (Giunciuglio, D. *et al*., Int J Cancer 63, 815-822 (1995)). One possible explanation for this is a lack of downstream signaling component for ErbB2 oncogenic transformation in MCF-10A cells.

Therefore, the issue of whether co-overexpression of Gab2 and ErbB2 may cause transformation of MCF-10A cells can be addressed. First, MCF-10A cells overexpressing both Gab2 and ErbB2 can be generated. MCF-10A overexpressing ErbB2 (MCF-10A-ErbB2) cells (kindly provided by Sam Lee, Beth Israel Deaconess Medical Center) can be infected with pBabe-puro virus alone, or with pBabe-puro virus containing Gab2 WT, or with pBabe-puro virus containing different Gab2 mutants such as ΔPI3K and ΔSHP2. MCF-10A-ErbB2 clones overexpressing a similar level of Gab2 WT and mutants can be screened by immunoblotting with Gab2 antibodies. At least two different clones expressing each Gab2 protein can be used for the subsequent studies. Next, the issue of whether MCF-10A-ErbB2-Gab2 cells become transformed can be examined by assaying their ability to grow in soft agar, form disorganized aggregates on Matrigel (inability to form acini), and induce tumor formation when injected into nude mice. If MCF-10A-ErbB2-Gab2 cells display all of the transforming phenotypes, this would suggest that overexpression of Gab2 together with ErbB2 overexpression can contribute to breast tumor formation *in vivo.*

To examine how Gab2 may cooperate with ErbB2 to cause the transformation of MCF-10A cells, one can test whether MCF-10A-ErbB2 clones expressing different Gab2 mutants (as discussed) can grow in soft agar, disrupt acini formation on Matrigel, and/or form tumor in mice. It would be expected that the Gab2-ΔPI3K mutant may be defective in these transformation assays, suggesting that Gab2 activation of PI-3K was important for breast carcinogenesis, consistent with the known role of PI-3K in oncogenic transformation (Cantley, L. & Neel, B., Proc Natl Acad Sci U S A 96, p4240-4245 (1999)). However, it cannot be ruled out that the Gab2-DSHP2 may be also defective in some of the transformation assays, considering that SHP-2 mainly functions as a positive signal transducer downstream of RTK including EGFR (Chen, B. *et al.,* Nat Genet 24, 296-299 (2000)).

### Example 15: Effects of overexpressing Gab2 on the growth/tumorigenicity of breast cancer cell MCF-7

Activation or inactivation of additional key molecules beside ErbB2 and Gab2 may be required for MCF-10A cells to become tumorigenic in mice. If overexpression of Gab2 and ErbB2 only causes transformation of MCF-10A cells *in vitro* (i.e., anchorage independent growth), but not tumorigenicity in mice, this would suggest that Gab2 may not be the only critical downstream target mediating the ErbB2 oncogenic signal.

To see whether overexpression of Gab2 can promote tumor formation, one can test whether overexpression of Gab2 will enhance the growth/or tumorigenicity of an already carcinogenic but relatively non-aggressive breast cancer cell MCF-7. MCF-7 cells express detectable level of ErbB2 (Cuello, M. *et al.,* Cancer Res 61, 4892-4900 (2001)) and Gab2. It has lower invasive property (Johnson, M. D. et al., Cancer Res 53, 873-877 (1993)) and forms tumors in nude mice with longer latency (Yue, W. & Brodie, A., J Steroid Biochem Mol Biol 44, 671-673 (1993)). A MCF-7 cell line that expresses Gab2 inducibly using the tetracycline (Tet)-off system has been generated. The Tet-off system has been used widely to achieve inducible expression of a gene of interest in cultured cells (Gossen, M. & Bujard, H., Proc Natl Acad Sci, USA 89,5547-5551 (1992)) as well as in mice (Huettner, C. S. et al., Nat. Genet. 24, 57-60 (2000)) upon decreasing or withdrawing tetracycline (Tet) from culture media and drinking water.

First, the issue of whether inducing the expression of Gab2 will enhance transforming properties of MCF-7 cells *in vitro* can be examined. For example, soft agar and transwell invasion assay using MCF-7 Gab2 cells in the absence or presence of Tet can be performed. If overexpression of Gab2 enhances MCF-7 transformation, one would expect that the formation of larger colonies in the absence of tetracycline (Gab2 is overexpressed) compared to in the presence of tetracycline, and/or that MCF-7 cells will invade through the Matrigel faster in the absence of Tet. Next, the tumorigenic property of MCF-7-Gab2 cells can be tested by injecting the cells into nude mice that have been maintained on Tet-containing water for several days. After injection, half of the injected mice can be still kept on Tet containing water and the other half of the mice can be on Tet free water. Typically, parental MCF-7 cells form visible tumors in nude mice about two months after inoculation. If tumors are observed earlier in mice on Tet free water, this would suggest that overexpression of Gab2 promotes enhanced breast tumor formation. To confirm that the tumors with early onset are due to Gab2 overexpression, Gab2 expression in these tumors can be examined by immunoblotting with Gab2 antibodies.

### Example 16: To investigate whether Gab2 is necessary and sufficient to promote breast cancer alone or in cooperation with the MMTV-Neu transgene

Although studies from Gab2 overexpression in breast epithelial cell lines *in vitro* suggest a role of Gab2 in breast carcinogenesis, the effect of Gab2 overexpression can be varied depending on different cell lines used in the studies. To test whether Gab2 expression is required for breast tumor formation and progress, mouse genetic approaches can be used.

### Example 17: Overexpression of Gab2 in a mammary gland using transgenic approach

The issue of whether Gab2 overexpression is sufficient to cause breast tumor in mice can be investigated. To address this question, transgenic mice overexpressing Gab2 specifically in the mammary gland can be generated. The transgenic construct, in which the Gab2 cDNA has two HA tags at its C-terminus, can be placed under the control of a WAP promoter, which is only active in mammary glands, to generate WAP-Gab2 transgenic mice. The generation of WAP-Gab2 transgenic mice in FvB strain lines can take about 6-8 months, during which western blot analysis can be used to check whether the founder transgenic lines specifically overexpress Gab2 in the mammary gland. Gab2 protein expressed from the transgene should run slower in SDS-PAGE because of the HA tags. By ten months, a large number of WAP-Gab2 transgenic mice that can be used to set up big breeding colonies for tumorigenic studies can be generated.

To assess whether Gab2 overexpression in a mammary gland is sufficient to cause a breast tumor, a large breeding colony can be established by mating the WAP-Gab2 transgenic mice with the wild type FvB mice. Since it is hard predict when breast tumor will form in WAP-Gab2 mice, these mice can be kept for the duration of their normal lifespan.

Since Gab2 may cooperate with ErbB2, the issue of whether Gab2 overexpression shortens the latency of breast tumor formation in MMTV-neu transgenic mice can be studied, in case WAP-Gab2 mice alone do not develop breast tumors. Therefore, a WAP-Gab2/MMTV-neu cross can be setup at the same time as the setup of the WAP-Gab2 mice interbreeding. Since MMTV-neu mice (obtained from Jackson ImmunoResearch Laboratory, West Grove, PA) typically develop mammary tumors around 6-7 month (Muller, W. J. et al., Cell 54,105-115 (1988)), the question of whether Gab2 overexpression in a mammary gland potentiates breast tumor formation induced by MMTV-neu transgene can be answered.

One possible outcome of this approach is that a WAP-Gab2 transgene alone may not cause a breast tumor, but it may potentiate breast tumor formation-induced MMTV-neu (i.e., it may significantly shorten the latency of the breast tumor onset). This would strongly imply that Gab2 overexpression in tumors is actively involved in the breast tumor progression by cooperation with one or more other oncogenes.

### Example 18: Analyzing a cross between Gab2 knockout mice and MMTV-neu transgenic mice.

Since published biochemical data suggest that Gab2 functions downstream of EGFR (ErbB1), the issue of whether Gab2 is required for mammary tumor induction in MMTV-neu mice can be investigated. To address this question, MMTV-neu mice can be crossed with the Gab2 knockout (-/-) mice. Gab2-/- mice are healthy, except that they have impaired allergy response. As a control, Gab2-/- mice can be crossed with MMTV-myc mice (developing mammary tumor about 8-10 months) since MMTV-neu and MMTV-myc induce mammary tumors through a different mechanism. MMTV-neu induction of breast tumors requires cyclin D1, MMTV-myc does not (Yu, Q. et al., Nature 411, 1017-1021 (2001)).

The Gab2-/- mice can be crossed with either MMTV-neu or myc mice. Gab2 +/:MMTV-neu and Gab2 +/-:MMTV-myc mice will result from these initial crosses (takes about three months). Gab2+/-:MMTV-neu mice can be interbred to generate Gab2-/:MMTV-neu and Gab2+/+:MMTV-neu mice, and Gab2+/-:MMTV-myc mice can be interbred to generate Gab2-/-:MMTV-myc and Gab2+/+:MMTV-myc mice. The progeny can be monitored for breast tumor formation in the progeny over a 14 -month period, which should allow Gab2-/-:MMTV-neu or Gab2-/-:MMTV-myc mice to show delayed onset of breast tumor formation.

If Gab2-/-:MMTV-neu mice show no or decreased breast tumor development within fourteen months, and Gab2-/-:MMTV-myc mice develop tumors with the same kinetics as Gab2+/+:MMTV-myc mice, this would suggest the genetic model that MMTV-Neu cause breast cancer via a Gab2-> cyclin D1 cascade. It is also possible that loss of Gab2 has no effects on the latency of breast tumor development in either MMTV-neu or MMTV-myc mice. This would suggest that breast tumor induction by the MMTV-neu or myc does not require normal endogenous levels of Gab2.

While this invention has been particularly shown and described with references to preferred embodiments thereof, it will be understood by those skilled in the art that various changes in form and details may be made therein without departing from the scope of the invention encompassed by the appended claims.
1. An isolated nucleic acid molecule comprising:
   a. Gab2 or a fragment, derivative or mutation thereof;
   b. the nucleic acid sequence of SEQ ID NO. 6;
   c. a sequence which encodes a polypeptide comprising amino acid sequence of SEQ ID NO. 5;
   d. a nucleic acid sequence with 90% sequence identity to SEQ ID NO. 6;
   e. a complementary strand of the sequence of (b), (c) or (d);
   f. RNA sequences transcribed from sequences (b), (c), (d) or (e), or a fragment or mutation thereof;
   g. DNA sequences that hybridize to the sequence of (b), (d) or (e).
2. An expression vector comprising the nucleic acid molecule according to Claim 1, or the nucleic acid molecule of Item 1 where the molecule has been mutated.
3. A host cell comprising the expression vector of Item 2.
4. The protein either encoded by the nucleic acid sequence of SEQ ID NO: 6 or comprising the amino acid sequence of SEQ ID NO: 5.
5. An antibody specific to the protein of Item 4.
6. A vector comprising the nucleic acid molecule of Item 1.
7. A probe comprising the nucleic acid molecule of Item 1.
8. A transgenic non-human mammal with a genome comprising a disruption of the Gab2 gene such that the mammal lacks or has reduced levels of functional Gab2 protein, and wherein the mammal exhibits an altered responsiveness to cytokine, growth factor, hormone or antigen stimulation.
9. A transgenic non-human mammal with a genome comprising an alteration of the Gab2 gene such that the mammal has increased levels of functional Gab2 protein, and wherein the mammal exhibits an altered responsiveness to cytokine, growth factor, hormone or antigen stimulation.
10. The transgenic non-human mammal of Item 8 or Item 9 wherein the mammal is a transgenic mouse.
11. The transgenic mouse of Item 8, Item 9 or Item 10 wherein the genome comprises a disruption of the Gab2 gene selected from the group consisting of a homozygous disruption and a heterozygous disruption.
12. Use of an agent which inhibits a Gab2 interaction with an associated protein for the manufacture of a medicament for preventing or treating a GAb2-mediated injury.
13. The use of Item 12 wherein the Gab2 interaction with an associated protein is in response to an extracellular stimulation.
14. The method of Item 13 wherein the extracellular stimulus is a cytokine, growth factor, hormone or antigen.
15. The use of Item 12, Item 13 or Item 14 wherein the Gab2-mediated injury is an allergic response, a neoplastic disease, or an immune disorder.
16. The use of any one of Items 12 to 15 wherein the agent is selected from the group consisting of proteins, polypeptides, antibodies, oligonucleotides, small molecules, natural product inhibitors, mutants of Gab2, and mutants of Gab2-associated molecules.
17. The use of any one of Items 12 to 15 wherein the agent is an oligonucleotide antisense to the nucleic acid sequence of Gab2, or antisense to a Gab2 homolog, fragment, complementary sequence, or mutant.
18. The use of any one of Items 12 to 15 wherein the agent is a mutant Gab2, or fragment thereof, which competes with Gab2 for interaction with its associated proteins.
19. The use of any one of Items 12 to 18 wherein tyrosyl phosphorylation of Gab2 is prevented by administration of the agent.
20. The use of any one of Items 12 to 19 wherein expression of Gab2 is inhibited or eliminated by administration of the agent.
21. The use of any one of Items 12 to 20 wherein the agent is administered for nasal, topical or systemic use.
22. The use of any one of Items 12 to 20 wherein the agent is an oligonucleotide administered as an insert in a gene therapy vector.
23. The use of any one of Items 12 to 22 wherein the associated protein is selected from the group consisting of p85, PI-3K, a protein containing a SH-2 domain, a protein containing a SH-3 domain, a protein containing a PH domain and a protein containing a WW domain.
24. The use of any one of Items 12 to 21 or Item 23 wherein the agent inhibits the response of mast cells to FceRI receptor stimulation by administration to the mast cells.
25. The use of any one of Items 12 to 21 or Item 23 wherein the Gab2-mediated injury is an allergic response and the agent inhibits said Gab2 interaction in response to an extracellular stimulus, thereby preventing activation of a Gab2-mediated signaling cascade.
26. The use of Item 24 or Item 25 wherein the response is degranulation, cytokine gene expression or anaphylaxis.
27. The use of any one of Items 12 to 20, Item 22 or Item 23 wherein the Gab2-mediated injury is a neoplastic disease and the agent prevents activation of a Gab2-mediated signaling cascade.
28. The use of Item 27 wherein the neoplastic disease is selected from the group consisting of leukemia, prostate cancer and breast cancer.
29. The use of any one of Items 12 to 20, Item 22 or Item 23 wherein the Gab2-mediated injury is breast cancer and the agent prevents activation of a Gab2-mediated signaling cascade.
30. The use of any one of Items 12 to 15 or Items 19 to 29 wherein the agent is a short, double-stranded RNA molecule or a short, double-stranded RNA/DNA combination, directed against Gab2 nucleic acid sequence for the purpose of decreasing or eliminating Gab2 gene expression.
31. A method of detecting upregulation of Gab2 product in a patient with a neoplastic disorder comprising testing a sample from a patient suspected of having a neoplastic disorder with the probe of Item 7.
32. A method of identifying a drug to be administered to treat a Gab2-mediated condition in a mammal in which the condition occurs, comprising:
   (a) producing a mouse that is a model of the condition;
   (b) administering to the mouse a drug to be assessed for its effectiveness in treating or preventing the condition; and
   (c) assessing the ability of the drug to treat or prevent the condition,
   wherein if the drug reduces the extent to which the condition is present or progresses, the drug is a drug to be administered to treat the condition.
33. The method of Item 32 wherein the mouse contains a genetic mutation causing a Gab2-mediated condition.
34. The method of Item 32 or Item 33 wherein the Gab2-mediated condition is an allergic response, a neoplastic disease, or an immune disorder.
35. Isolated RNA that mediates RNA interference of Gab2 mRNA.
36. Isolated RNA of Item 35 that comprises a terminal 3' hydroxyl group.
37. Isolated RNA of Item 35 or Item 36 which is chemically synthesized RNA or an analog of a naturally occurring RNA.
38. An analog of isolated RNA of Item 35, Item 36 or Item 37 wherein the analog differs from the RNA of Gab2 by the addition, deletion, substitution or alteration of one or more nucleotides.
39. Isolated RNA that inactivates the Gab2 gene by transcriptional silencing.
40. A method of mediating RNA interference of mRNA of the Gab2 gene in a cell or organism comprising:
   a. introducing RNA of sufficient length which targets the mRNA of the Gab2 gene for degradation into the cell or organism; and
   b. maintaining the cell or organism produced in (a) under conditions under which degradation of the mRNA occurs, thereby mediating RNA interference of the mRNA of the gene in the cell or organism.
41. The method of Item 40 wherein the RNA of (a) is a chemically synthesized RNA or an analog of naturally occurring RNA.
42. A method of mediating RNA interference of mRNA of the Gab2 gene in a cell or organism in which RNA interference occurs, comprising:
   a. combining double-stranded RNA that corresponds to a sequence of the Gab2 gene with a soluble extract that mediates RNA interference, thereby producing a combination;
   b. maintaining the combination produced in (a) under conditions under which the double-stranded RNA is processed to RNA of sufficient length, thereby producing processed RNA of sufficient length;
   c. isolating the RNA of sufficient length produced in (b);
   d. introducing RNA isolated in (c) into the cell or organism; and
   e. maintaining the cell or organism produced in (d) under conditions under which degradation of mRNA of the Gab2 gene occurs, thereby mediating RNA interference of the mRNA of the Gab2 gene in the cell or organism.
43. The method of Item 42 wherein the processed RNA of step (b) is of from about 21 to 23 nucleotides.
44. A method of mediating RNA interference ofmRNA of the Gab2 gene in a cell or organism in which RNA interference occurs, comprising:
   (a) introducing into the cell or organism RNA of sufficient length that mediates RNA interference of mRNA of the Gab2 gene, thereby producing a cell or organism that contains the RNA; and
   (b) maintaining the cell or organism that contains the RNA under conditions under which RNA interference occurs, thereby mediating RNA interference of mRNA of the Gab2 gene in the cell or organism.
45. The use of an agent for the manufacture of a medicament for treating a disease or condition associated with the presence of a Gab2 protein in an individual wherein the agent comprises RNA of sufficient length that targets the mRNA of the Gab2 gene for degradation.
46. The use of Item wherein RNA of sufficient length is chemically synthesized or an analog of RNA that mediates RNA interference.
47. The use of Item 45 or Item 46 wherein the agent is used in a method according to any one of Claims 40 to 44.
48. The use of any one of Items 45 to 47 wherein the agent comprises the indicated RNA of any one of Items 35 to 39.

The claims of the present application are as follows:

### Annex to the application documents - subsequently filed sequences listing

SEQUENCE LISTING

<110> Beth Israel Deaconess Medical Center, Inc.

<120> Gab2 (p97) Gene and Methods of Use
  Thereof

<130> 16053div1:nak

<140> 05076966.0
<141> 2001-10-26

<150> 60/243,495
<151> 2000-10-26

<160> 54

<170> FastSEQ for Windows Version 4.0

<210> 1
<211> 6
<212> PRT
<213> Unknown

<220>
<223> Murine

<400> 1
Lys Ala Lys Pro Thr Pro
 1               5

<210> 2
<211> 6
<212> PRT
<213> Unknown

<220>
<223> Murine

<400> 2
Thr Val Ile Asp Glu Leu
 1               5

<210> 3
<211> 21
<212> DNA
<213> Artificial Sequence

<220>
<223> primer sequence

<400> 3
ccttgacctg agaaacaaca c                                                21

<210> 4
<211> 6
<212> PRT
<213> Unknown

<220>
<223> Murine

<400> 4
Leu Asp Leu Arg Asn Asn
1               5


<210> 5
<211> 666
<212> PRT
<213> Unknown

<220>
<223> Murine

<400> 5
Met Ser Gly Gly Gly Gly Asp Asp Val Val Cys Thr Gly Trp Leu Arg
1               5                   10                  15
Lys Ser Pro Pro Glu Lys Lys Leu Arg Arg Tyr Ala Trp Lys Lys Arg
            20                  25                  30
Trp Phe Ile Leu Arg Ser Gly Arg Met Ser Gly Asp Pro Asp Val Leu
        35                  40                  45
Glu Tyr Tyr Lys Asn Glu His Ser Lys Lys Pro Leu Arg Ile Ile Asn
    50                  55                  60
Leu Asn Leu Cys Glu Gln Val Asp Ala Gly Leu Thr Phe Asn Lys Lys
65                  70                  75                  80
Glu Leu Gln Asp Ser Phe Val Phe Asp Ile Lys Thr Ser Glu Arg Thr
                85                  90                  95
Phe Tyr Leu Val Ala Glu Thr Glu Ala Asp Met Asn Lys Trp Val Gln
            100                 105                 110
Ser Ile Cys Gln Ile Cys Gly Phe Asn Gln Ala Glu Glu Ser Thr Asp
        115                 120                 125
Ser Leu Arg Asn Leu Ser Ser Ala Ser His Gly Pro Arg Ser Ser Pro
    130                 135                 140
Ala Glu Phe Ser Ser Ser Gln His Leu Leu Arg Glu Arg Lys Ser Ser
145                 150                 155                 160
Ala Pro Ser His Ser Ser Gln Pro Thr Leu Phe Thr Phe Glu Pro Pro
                165                 170                 175
Val Ser Ser His Met Gln Pro Thr Leu Ser Thr Ser Ala Pro Gln Glu
            180                 185                 190
Tyr Leu Tyr Leu His Gln Cys Ile Ser Arg Arg Thr Glu Asn Ala Arg
        195                 200                 205
Ser Ala Ser Phe Ser Gln Gly Thr Arg Gln Lys Ser Asp Thr Ala Val
    210                 215                 220
Gln Lys Leu Ala Gln Ser Asn Gly His Cys Ile Asn Gly Val Gly Gly
225                 230                 235                 240
Gln Val His Gly Phe Tyr Ser Leu Pro Lys Pro Ser Arg His Asn Thr
                245                 250                 255
Glu Phe Lys Asp Ser Thr Tyr Asp Leu Pro Arg Ser Leu Ala Ser His
            260                 265                 270
Gly His Thr Lys Ser Ser Leu Thr Gly Ser Glu Thr Asp Asn Glu Asp
        275                 280                 285
Val Tyr Thr Phe Lys Met Pro Ser Asn Thr Leu Cys Arg Glu Leu Gly
    290                 295                 300
Asp Leu Leu Val Asp Asn Met Asp Val Pro Thr Thr Pro Leu Ser Ala
305                 310                 315                 320
Tyr Gln Ile Pro Arg Thr Phe Thr Leu Asp Lys Asn His Asn Ala Met
                325                 330                 335
Thr Val Ala Thr Pro Gly Asp Ser Ala Ile Ala Pro Pro Pro Arg Pro
            340                 345                 350
Pro Lys Pro Ser Gln Ala Glu Thr Ser Gln Trp Gly Ser Ile Gln Gln
        355                 360                 365
Arg Pro Pro Ile Ser Glu Asn Ser Arg Ser Val Ala Ala Thr Ile Pro
    370                 375                 380
Arg Arg Asn Thr Leu Pro Ala Met Asp Asn Ser Arg Leu His Arg Ala
385                 390                 395                 400
Ser Ser Cys Glu Thr Tyr Glu Tyr Pro Ala Arg Gly Ser Gly Glu Ser
                405                 410                 415
Ala Ser Trp Ser Ala Glu Pro Pro Gly Lys Thr Ala Val Gly Arg Ser

420                     425                     430
Asn Ser Ala Ser Ser Asp Asp Asn Tyr Val Pro Met Asn Pro Gly Ser
        435                     440                     445
Ser Thr Leu Leu Ala Met Glu Arg Pro Gly Asp Asn Ser Gln Ser Val
    450                     455                     460
Tyr Ile Pro Met Ser Pro Gly Pro His His Phe Asp Pro Leu Gly Tyr
465                     470                     475                     480
Pro Ser Thr Ala Leu Pro Ile His Arg Gly Pro Ser Arg Gly Ser Glu
                485                     490                     495
Ile Gln Pro Pro Pro Val Asn Arg Asn Leu Lys Pro Asp Arg Lys Ala
            500                     505                     510
Lys Pro Thr Pro Leu Asp Leu Arg Asn Asn Thr Val Ile Asp Glu Leu
        515                     520                     525
Pro Phe Lys Ser Pro Val Thr Lys Ser Trp Ser Arg Ile Asn Ser His
    530                     535                     540
Thr Phe Asn Ser Ser Ser Ser Gln Tyr Cys Arg Pro Ile Ser Thr Gln
545                     550                     555                     560
Ser Ile Thr Ser Thr Asp Ser Gly Asp Ser Glu Glu Asn Tyr Val Pro
                565                     570                     575
Met Gln Asn Pro Val Ser Ala Ser Pro Val Pro Ser Gly Thr Asn Ser
            580                     585                     590
Pro Ala Pro Lys Lys Ser Thr Gly Ser Val Asp Tyr Leu Ala Leu Asp
        595                     600                     605
Phe Gln Pro Gly Ser Pro Ser Pro His Arg Lys Pro Ser Thr Ser Ser
    610                     615                     620
Val Thr Ser Asp Glu Lys Val Asp Tyr Val Gln Val Asp Lys Glu Lys
625                     630                     635                     640
Thr Gln Ala Leu Gln Asn Thr Met Gln Glu Trp Thr Asp Val Arg Gln
                645                     650                     655
Ser Ser Glu Pro Ser Lys Gly Ala Lys Leu
            660                     665

<210> 6
<211> 1965
<212> DNA
<213> Unknown

<220>
<223> Murine

<400> 6
atgagcggcg gcggcggcga cgacgtggtg tgtaccggct ggctgaggaa atcgcctccc 60
gagaagaagt tgaggcgcta tgcctggaag aaacgctggt ttatacttcg gagtggccga 120
atgagtggag atccagatgt tctggaatac tacaagaatg agcactccaa gaaacccctg 180
cggatcatca acctgaactt gtgtgagcag gtggatgcag gcctgacctt caacaagaaa 240
gagctgcagg atagttttgt gtttgatatc aagaccagcg agcgcacatt ttacctggtg 300
gctgagacag aggctgacat gaataagtgg gtccagagca tctgccagat ctgcggcttc 360
aatcaggctg aagagagcac agactccctg aggaaccttt cttcagccag tcatggtccc 420
cgctcttctc cagctgagtt cagctccagt cagcacctgc tccgagaacg gaagtcctca 480
gcccttcaca ctctagccag cctactttat tcacgtttga gccccctgtg tcaagccaca 540
tgcagcctac ctgtccacca gtgcacctca ggagtatctc tacttgcacc agtgcataag 600
cagaaggaca gaaaatgcaa ggagtgccag cttctctcag ggcacccggc agaagagtga 660
tacagctgtg caaaaacttg cccagagcaa tggacactgt atcaacggcg tcggaggtca 720
agtccatggc ttctatagcc ttcccaagcc aagccgacac aatacagaat tcaaagacag 780
tacttatgat ctcccacgga gcctggcttc ccatggccac accaagagca gcctcacagg 840
gtctgagact gataacgagg atgtgtacac cttcaagatg cccagcaaca ccctgtgtcg 900
ggaacttgga gacctccttg tggacaatat ggatgtccca accactcctc tctcagccta 960
ccagatccct agaacattca cactggacaa gaaccacaat gccatgacag tggccactcc 1020
tggagattca gccatagctc cccccacccg gccacccaag ccaagtcagg cagaaacatc 1080
tcaatggggc agcattcagc aaagacctcc aatcagcgaa aatagcagat ctgtagctgc 1140
tactatcccc aggcgcaata ccctccctgc aatggacaac agccgactcc atcgagcttc 1200
ttcctgtgag acctacgagt acccggcacg aggcagtggg gaaagtgcca gctggtctgc 1260
tgaacctcca ggaaagactg ccgtaggtcg atcaaatagt gccagctctg atgacaacta 1320
cgtgcccatg aacccaggtt cttctaccct gctggctatg gaacgaccag gggacaactc 1380
ccagagtgtc tacatcccca tgagcccagg accccatcac tttgacccac ttggctaccc 1440
gccacagccc ttcctattca cagaggcccc agccgaggaa gtgagatcca gccacccccg 1500
gtcaaccgaa acctcaagcc tgacagaaaa gcaaagccaa caccccttga cctgagaaac 1560

aacactgtca tcgatgacct gcccttcaag tcacctgtca ccaagtcttg gtccaggatc 1620
aacagccaca cctttaactc cagttcctcc cagtactgcc gtcactatgt ccctatgcaa 1680
aacccagtat ctgcatcccc tgttcccagt ggcactaaca gcccagctcc aaagaagagt 1740
actggcagtg tggattatct cgccctggac ttccagccgg gctccccaag ccctcaccgc 1800
aagccatcca catcatctgt cacatcagat gagaaggtag actatgtcca agtggataaa 1860
gagaagaccc aggccctgca gaacaccatg caggagtgga cagatgtgcg ggcagtcctc 1920
cgaaccttcc aagggtgcca agctgtaatg aagggggcca ccaag 1965

<210> 7
<211> 12
<212> PRT
<213> Unknown

<220>
<223> Murine

<400> 7
Gln Leu Glu Glu Asp Tyr Tyr Gly Leu Ser Lys Gly
1               5                   10

<210> 8
<211> 7
<212> PRT
<213> Unknown

<220>
<223> Murine

<400> 8
Thr Gln Ala Leu Gln Asn Thr
1               5

<210> 9
<211> 11
<212> PRT
<213> Unknown

<220>
<223> Murine

<400> 9
Asp Ser Thr Tyr Asp Leu Pro Arg Ser Leu Ala
1               5                   10

<210> 10
<211> 9
<212> PRT
<213> Unknown

<220>
<223> Murine

<400> 10
Pro Ser Arg His Asn Thr Glu Phe Lys
1               5

<210> 11
<211> 5
<212> PRT
<213> Unknown

<220>
<223> Murine

<400> 11
Val Gln Val Asp Lys
1               5


<210> 12
<211> 11
<212> PRT
<213> Unknown

<220>
<223> Murine

<400> 12
Glu Leu Gln Asp Ser Phe Val Phe Asp Ile Lys
1               5                   10


<210> 13
<211> 17
<212> PRT
<213> Unknown

<220>
<223> Murine

<400> 13
Ala Lys Pro Thr Pro Leu Asp Leu Arg Asn Asn Thr Val Ile Asp Glu
1               5                   10                  15
Leu


<210> 14
<211> 17
<212> PRT
<213> Unknown

<220>
<223> Murine

<400> 14
Ser Ser Leu Thr Gly Ser Glu Thr Asp Asn Glu Asp Val Tyr Thr Phe
1               5                   10                  15
Lys


<210> 15
<211> 4
<212> PRT
<213> Unknown

<220>
<223> Murine

<400> 15
Tyr Lys Asn Glu
1


<210> 16
<211> 4
<212> PRT
<213> Unknown

<220>
<223> Murine

<400> 16
Tyr Ser Leu Pro
1

<210> 17
<211> 4
<212> PRT
<213> Unknown

<220>
<223> Murine

<400> 17
Tyr Asp Leu Pro
1

<210> 18
<211> 4
<212> PRT
<213> Unknown

<220>
<223> Murine

<400> 18
Tyr Gln Ile Pro
1

<210> 19
<211> 4
<212> PRT
<213> Unknown

<220>
<223> Murine

<400> 19
Tyr Glu Tyr Pro
1

<210> 20
<211> 4
<212> PRT
<213> Unknown

<220>
<223> Murine

<400> 20
Tyr Val Pro Met
1

<210> 21
<211> 4
<212> PRT
<213> Unknown

<220>
<223> Murine

<400> 21
Tyr Ile Pro Met

1


<210> 22
<211> 4
<212> PRT
<213> Unknown

<220>
<223> Murine

<400> 22
Tyr Leu Ala Leu
1


<210> 23
<211> 4
<212> PRT
<213> Unknown

<220>
<223> Murine

<400> 23
Tyr Val Gln Val
1


<210> 24
<211> 4
<212> PRT
<213> Unknown

<220>
<223> Murine

<400> 24
Lys Ser Pro Pro
1


<210> 25
<211> 118
<212> PRT
<213> Unknown

<220>
<223> Murine

<400> 25
Met Ser Gly Gly Gly Gly Asp Asp Val Val Cys Thr Gly Trp Leu Arg
1               5                   10                  15
Lys Ser Pro Pro Glu Lys Lys Leu Arg Arg Tyr Ala Trp Lys Lys Arg
            20                  25                  30
Trp Phe Ile Leu Arg Ser Gly Arg Met Ser Gly Asp Pro Asp Val Leu
        35                  40                  45
Glu Tyr Tyr Lys Asn Glu His Ser Lys Lys Pro Leu Arg Ile Ile Asn
    50                  55                  60
Leu Asn Leu Cys Glu Gln Val Asp Ala Gly Leu Thr Phe Asn Lys Lys
65                  70                  75                  80
Glu Leu Gln Asp Ser Phe Val Phe Asp Ile Lys Thr Ser Glu Arg Thr
                85                  90                  95
Phe Tyr Leu Val Ala Glu Thr Glu Ala Asp Met Asn Lys Trp Val Gln
            100                 105                 110
Ser Ile Cys Gln Ile Cys
        115

<210> 26
<211> 115
<212> PRT
<213> Unknown

<220>
<223> Murine

<400> 26
Met Ser Gly Gly Glu Val Val Cys Ser Gly Trp Leu Arg Lys Ser Pro
1               5                   10                  15
Pro Glu Lys Lys Leu Lys Arg Tyr Ala Trp Lys Lys Arg Trp Phe Val
            20                  25                  30
Leu Arg Ser Gly Arg Leu Thr Gly Asp Pro Asp Val Leu Glu Tyr Tyr
        35                  40                  45
Lys Asn Asp His Ala Lys Lys Pro Ile Arg Ile Ile Asp Leu Asn Leu
    50                  55                  60
Cys Gln Gln Val Asp Ala Gly Leu Thr Phe Asn Lys Lys Glu Phe Glu
65                  70                  75                  80
Asn Ser Tyr Ile Phe Asp Ile Asn Thr Ile Asp Arg Ile Phe Tyr Leu
                85                  90                  95
Val Ala Asp Ser Glu Glu Glu Met Asn Lys Trp Val Arg Cys Ile Cys
            100                 105                 110
Asp Ile Cys
        115


<210> 27
<211> 112
<212> PRT
<213> Unknown

<220>
<223> Murine

<400> 27
Met Asp Arg Thr Phe Tyr Glu Gly Trp Leu Ile Lys Ser Pro Pro Thr
1               5                   10                  15
Lys Arg Ile Trp Arg Ala Arg Trp Arg Arg Arg Tyr Phe Thr Leu Lys
            20                  25                  30
Gln Gly Glu Ile Pro Glu Gln Phe Cys Leu Glu Tyr Tyr Thr Asp His
        35                  40                  45
Asn Cys Arg Lys Leu Lys Gly Val Ile Asp Leu Asp Gln Cys Glu Gln
    50                  55                  60
Val Asp Cys Gly Leu Arg Leu Glu Asn Arg Lys Gln Lys Phe Gln Tyr
65                  70                  75                  80
Met Phe Asp Ile Lys Thr Pro Lys Arg Thr Tyr Tyr Leu Ala Ala Glu
                85                  90                  95
Thr Glu Ala Asp Met Arg Asp Trp Val Asn Cys Ile Cys Gln Val Cys
            100                 105                 110


<210> 28
<211> 4
<212> PRT
<213> Unknown

<220>
<223> Murine

<400> 28
Leu Glu Tyr Tyr
1


<210> 29
<211> 83

<212> PRT
<213> Unknown

<220>
<223> Murine

<400> 29
Pro Met Asn Pro Asn Ser Pro Pro Arg Gln His Ser Ser Ser Phe Thr
1               5                   10                  15
Glu Pro Ile Gln Glu Ala Asn Tyr Val Pro Met Thr Pro Gly Thr Phe
            20                  25                  30
Asp Phe Ser Ser Phe Gly Met Gln Val Pro Pro Pro Ala His Met Gly
        35                  40                  45
Phe Arg Ser Ser Pro Lys Thr Pro Pro Arg Arg Pro Val Pro Val Ala
    50                  55                  60
Gln Cys Glu Pro Pro Pro Val Asp Arg Asn Leu Lys Pro Asp Arg Lys
65                  70                  75                  80
Val Lys Pro

<210> 30
<211> 4
<212> PRT
<213> Unknown

<220>
<223> Murine

<400> 30
Pro Met Asn Pro
1

<210> 31
<211> 4
<212> PRT
<213> Unknown

<220>
<223> Murine

<400> 31
Pro Pro Pro Val
1

<210> 32
<211> 8
<212> PRT
<213> Unknown

<220>
<223> Murine

<400> 32
Arg Asn Leu Lys Pro Asp Arg Lys
1               5

<210> 33
<211> 72
<212> PRT
<213> Unknown

<220>
<223> Murine

<400> 33
Pro Met Asn Pro Gly Ser Ser Thr Leu Leu Ala Met Glu Arg Pro Gly
1               5                   10                  15
Asp Asn Ser Gln Ser Val Tyr Ile Pro Met Ser Pro Gly Pro His His
            20                  25                  30
Phe Asp Pro Leu Gly Tyr Pro Ser Thr Ala Leu Pro Ile His Arg Gly
        35                  40                  45
Pro Ser Arg Gly Ser Glu Ile Gln Pro Pro Pro Val Asn Arg Asn Leu
    50                  55                  60
Lys Pro Asp Arg Lys Ala Lys Pro
65                  70


<210> 34
<211> 4
<212> PRT
<213> Unknown

<220>
<223> Murine

<400> 34
Tyr Cys Arg Pro
1


<210> 35
<211> 4
<212> PRT
<213> Unknown

<220>
<223> Murine

<400> 35
Tyr Leu Tyr Leu
1


<210> 36
<211> 9
<212> PRT
<213> Unknown

<220>
<221> VARIANT
<222> 1
<223> Xaa = Gln or Glu or Pro

<221> VARIANT
<222> 2
<223> Xaa = Ile or Ser

<221> VARIANT
<222> 3
<223> Xaa = Leu or Arg

<221> VARIANT
<222> 4
<223> Xaa = Asp or His

<221> VARIANT
<222> 5
<223> Xaa = Asn or Lys

<223> Murine

<400> 36

Xaa Xaa Xaa Xaa Xaa Thr Glu Phe Lys
1               5

<210> 37
<211> 4
<212> PRT
<213> Unknown

<220>
<223> Murine

<221> VARIANT
<222> 2, 3
<223> Xaa = Any Amino Acid

<400> 37
Pro Xaa Xaa Pro
1

<210> 38
<211> 4
<212> PRT
<213> Unknown

<220>
<223> Murine

<400> 38
Tyr Val Asn Thr
1

<210> 39
<211> 4
<212> PRT
<213> Unknown

<220>
<223> Murine

<400> 39
Tyr Ser Asn Arg
1

<210> 40
<211> 4
<212> PRT
<213> Unknown

<220>
<223> Murine

<400> 40
Tyr Ile Pro Ile
1

<210> 41
<211> 4
<212> PRT
<213> Unknown

<220>
<223> Murine

<400> 41
Tyr Asp Thr Pro
1

<210> 42
<211> 4
<212> PRT
<213> Unknown

<220>
<223> Murine

<400> 42
Tyr Asp Ile Pro
1

<210> 43
<211> 4
<212> PRT
<213> Unknown

<220>
<223> Murine

<400> 43
Tyr Tyr Asn Val
1

<210> 44
<211> 4
<212> PRT
<213> Unknown

<220>
<223> Murine

<400> 44
Tyr Thr Asn Ala
1

<210> 45
<211> 4
<212> PRT
<213> Unknown

<220>
<223> Murine

<400> 45
Tyr Asp Phe Met
1

<210> 46
<211> 4
<212> PRT
<213> Unknown

<220>
<223> Murine

<400> 46
Tyr Phe Asp Leu
1

<210> 47
<211> 4
<212> PRT
<213> Unknown

<220>
<223> Murine

<400> 47
Tyr Arg Ser Val
1


<210> 48
<211> 4
<212> PRT
<213> Unknown

<220>
<223> Murine

<400> 48
Tyr Lys Asn Asp
1


<210> 49
<211> 4
<212> PRT
<213> Unknown

<220>
<223> Murine

<400> 49
Tyr Leu Leu Leu
1


<210> 50
<211> 4
<212> PRT
<213> Unknown

<220>
<223> Murine

<400> 50
Tyr Asp Ser Pro
1


<210> 51
<211> 4
<212> PRT
<213> Unknown

<220>
<223> Murine

<400> 51
Tyr Asn Leu Pro
1


<210> 52

<211> 4
<212> PRT
<213> Unknown

<220>
<223> Murine

<400> 52
Tyr Cys Ile Pro
1


<210> 53
<211> 4
<212> PRT
<213> Unknown

<220>
<223> Murine

<400> 53
Tyr Leu Asp Leu
1


<210> 54
<211> 4
<212> PRT
<213> Unknown

<220>
<223> Murine

<400> 54
Tyr Val Val Val
1

## Claims

1. A method for preventing or treating a GAb2-mediated injury, comprising administering an agent which inhibits a Gab2 interaction with an associated protein.

2. The method of Claim 1 wherein a) the Gab2 interaction with an associated protein is in response to an extracellular stimulation, and optionally the extracellular stimulus is a cytokine, growth factor, hormone or antigen, or
b) the Gab2-mediated injury is an allergic response, a neoplastic disease, or an immune disorder, or
c) the agent is selected from the group consisting of proteins, polypeptides, antibodies, oligonucleotides, small molecules, natural product inhibitors, mutants of Gab2, and mutants of Gab2-associated molecules, or
d) the agent is an oligonucleotide antisense to the nucleic acid sequence of Gab2, or antisense to a Gab2 homolog, fragment, complementary sequence, or mutant, or
e) the agent is a mutant Gab2, or fragment thereof, which competes with Gab2 for interaction with its associated proteins, or
f) tyrosyl phosphorylation of Gab2 is prevented by administration of the agent, or
g) expression of Gab2 is inhibited or eliminated by administration of the agent, or
h) the agent is administered for nasal, topical or systemic use, or
i) the agent is an oligonucleotide administered as an insert in a gene therapy vector, or
j) the associated protein is selected from the group consisting of p85, PI-3K, a protein containing a SH-2 domain, a protein containing a SH-3 domain, a protein containing a PH domain and a protein containing a WW domain, or
k) the agent inhibits the response of mast cells to FceRI receptor stimulation by administration to the mast cells, or
l) the Gab2-mediated injury is an allergic response and the agent inhibits said Gab2 interaction in response to an extracellular stimulus, thereby preventing activation of a Gab2-mediated signaling cascade, and optionally the response is degranulation, cytokine gene expression or anaphylaxis, or
m) the Gab2-mediated injury is a neoplastic disease and the agent prevents activation of a Gab2-mediated signaling cascade, or
n) the neoplastic disease is selected from the group consisting of leukemia, prostate cancer and breast cancer, or o) the Gab2-mediated injury is breast cancer and the agent prevents activation of a Gab2-mediated signaling cascade, or
p) the agent is a short, double-stranded RNA molecule or a short, double-stranded RNA/DNA combination, directed against Gab2 nucleic acid sequence for the purpose of decreasing or eliminating Gab2 gene expression.

3. A method of detecting upregulation of Gab2 product in a patient with a neoplastic disorder comprising testing a sample from a patient suspected of having a neoplastic disorder with the probe comprising an isolated nucleic acid molecule comprising:
1. Gab2 or a fragment, derivative or mutation thereof;
2. the nucleic acid sequence of SEQ ID NO. 6;
3. a sequence which encodes a polypeptide comprising amino acid sequence of SEQ ID NO. 5;
4. a nucleic acid sequence with 90% sequence identity to SEQ ID NO. 6;
5. a complementary strand of the sequence of (b), (c) or (d);
6. RNA sequences transcribed from sequences (b), (c), (d) or (e), or a fragment or mutation thereof;
7. DNA sequences that hybridize to the sequence of (b), (d) or (e).

4. A method of identifying a drug to be administered to treat a Gab2-mediated condition in a mammal in which the condition occurs, comprising:
(a) producing a mouse that is a model of the condition;
(b) administering to the mouse a drug to be assessed for its effectiveness in treating or preventing the condition; and
(c) assessing the ability of the drug to treat or prevent the condition,
wherein if the drug reduces the extent to which the condition is present or progresses, the drug is a drug to be administered to treat the condition.

5. The method of Claim 4 wherein a) the mouse contains a genetic mutation causing a Gab2-mediated condition, or
b) the Gab2-mediated condition is an allergic response, a neoplastic disease, or an immune disorder.

6. Isolated RNA that mediates RNA interference of Gab2 mRNA.

7. Isolated RNA of Claim 6, a) that comprises a terminal 3' hydroxyl group,
or b) which is chemically synthesized RNA or an analog of a naturally occurring RNA,
or c) wherein the analog differs from the RNA of Gab2 by the addition, deletion, substitution or alteration of one or more nucleotides.

8. (Original) Isolated RNA that inactivates the Gab2 gene by transcriptional silencing.

9. A method of mediating RNA interference of mRNA of the Gab2 gene in a cell or organism comprising:
a. introducing RNA of sufficient length which targets the mRNA of the Gab2 gene for degradation into the cell or organism; and
b. maintaining the cell or organism produced in (a) under conditions under which degradation of the mRNA occurs, thereby mediating RNA interference of the mRNA of the gene in the cell or organism.

10. The method of Claim 9 wherein the RNA of (a) is a chemically synthesized RNA or an analog of naturally occurring RNA.

11. A method of mediating RNA interference of mRNA of the Gab2 gene in a cell or organism in which RNA interference occurs, comprising:
a. combining double-stranded RNA that corresponds to a sequence of the Gab2 gene with a soluble extract that mediates RNA interference, thereby producing a combination;
b. maintaining the combination produced in (a) under conditions under which the double-stranded RNA is processed to RNA of sufficient length, thereby producing processed RNA of sufficient length;
c. isolating the RNA of sufficient length produced in (b);
d. introducing RNA isolated in (c) into the cell or organism; and
e. maintaining the cell or organism produced in (d) under conditions under which degradation of mRNA of the Gab2 gene occurs, thereby mediating RNA interference of the mRNA of the Gab2 gene in the cell or organism.

12. The method of Claim 11 wherein the processed RNA of step (b) is of from about 21 to 23 nucleotides.

13. A method of mediating RNA interference of mRNA of the Gab2 gene in a cell or organism in which RNA interference occurs, comprising:
(a) introducing into the cell or organism RNA of sufficient length that mediates RNA interference ofmRNA of the Gab2 gene, thereby producing a cell or organism that contains the RNA; and
(b) maintaining the cell or organism that contains the RNA under conditions under which RNA interference occurs, thereby mediating RNA interference of mRNA of the Gab2 gene in the cell or organism.

14. A method for treating a disease or condition associated with the presence of a Gab2 protein in an individual wherein the agent comprises RNA of sufficient length that targets the mRNA of the Gab2 gene for degradation.

15. The method of Claim 14 wherein a) RNA of sufficient length is chemically synthesized or an analog of RNA that mediates RNA interference, or
b) the agent is used in a method of mediating RNA interference of mRNA of the Gab2 gene in a cell or organism comprising:
a. introducing RNA of sufficient length which targets the mRNA of the Gab2 gene for degradation into the cell or organism; and
b. maintaining the cell or organism produced in (a) under conditions under which degradation of the mRNA occurs, thereby mediating RNA interference of the mRNA of the gene in the cell or organism, or
c) the agent comprises isolated RNA that mediates RNA interference of Gab2 mRNA.
